# EUROPEAN PATENT APPLICATION

(11) **EP 4 353 319 A2**
(43) Date of publication of application: **17.04.2024**
(21) Application number: 23212108.7
(22) Date of filing: 25.09.2017
(51) Int. Cl.: A61P 35/00

(54) **METHODS OF ADOPTIVE CELL THERAPY**

(30) Priority: 28.09.2016 US 201662401040 P
(62) Divisional of application: 17857260.8
(71) Applicant: Atossa Therapeutics, Inc., Seattle, WA 98104 (US)
(72) Inventor: QUAY, Steven, C., Seattle (US)
(74) Representative: Titmus, Craig Edward

(57) **Abstract**

The present invention relates to compositions and transpapillary methods of adoptive cell therapy for the treatment of subjects having or at risk of having breast disorders.

## Description

### CROSS-REFERENCE TO RELATED APPLICATION

This application claims the benefit of U.S. Application No. 62/401040, filed September 28, 2016, which is incorporated herein by reference in its entirety.

### STATEMENT REGARDING SEQUENCE LISTING

The sequence listing associated with this application is provided in XML format in lieu of a paper copy and is hereby incorporated by reference into the specification. The name of the XML file containing the sequence listing is 438558-763202_763EPD1_SL.xml. The XML file is 29,127 bytes; was created on January 25, 2024; and is being submitted electronically.

### BACKGROUND

Adoptive cell therapy using engineered cells expressing various recombinant receptors, such as chimeric antigen receptors (CARs) have been developed for the treatment of cancers. Adoptive cell therapy has been demonstrated to have some success in treating blood-borne cancers, prominently, with the use of CD19 CARs in leukemias, and indications in patients with lymphoma and myeloma are being explored. Treatment of solid tumors is much more problematic for a variety of reasons. Presence of physical barriers makes transendothelial crossing and the targeting and infiltration of solid tumors (i.e., intratumoral migration) of such cells difficult.

Additionally, the presence of hostile tumor microenvironment has resulted in poor access of the administered cells to target cells and in tumor escape. The tumor microenvironment includes conditions such as high tissue pressure, hypoxia, nutritional starvation (for example due to reduced glucose and other metabolites), increased lactate generation and resulting acidosis, increased infiltration of regulatory CD4+ T-cells (T-regs), myeloid derived suppressor cells (MDSCs), and tumor associated macrophages (TAMs), presence of immunosuppressive cytokines (such as IL-10 and TGF-β), and expression of ligands targeted to immune suppressive receptors expressed by activated T-cells (such as CTLA4 and PD-1), and reduced T-cell proliferation, which helps to create immunosuppressive microenvironment allowing tumors to protect themselves from immune recognition, maintain tolerance, and evade elimination. Other challenges occasionally observed in subjects with solid tumors are "cytokine storms", also known as cytokine release syndrome (CRS), macrophage activation syndrome (MAS), tumor lysis syndrome (TLS), neurotoxicity, host immune response to transferred cells, which result in uncontrolled released of cytokines from synchronously activated and rapidly proliferating CAR-T cells and macrophages, albeit seen less often in subjects with solid tumors compared to those with blood cancers. Adverse events such as CRS observed post-adoptive cell therapy for example, CAR-T cell therapy, highlights the need for caution while using CAR-T cells. An extreme example of CRS-related adverse events includes the death of a colon cancer subject preconditioned with cyclophosphamide 5 days after infusion of CAR-T cells targeting the ERBB2 (HER-2/neu) antigen (Morgan et al. Molecular therapy: J. Am. Soc. of Gene Therapy. 28 (4): 843-51). In that case, toxicity led to a clinically significant release of pro-inflammatory cytokines, pulmonary toxicity, multi-organ failure and eventual patient death. This cytokine storm is thought to be due to CAR-T cell cytotoxicity against normal lung epithelial cells, which are known to express low levels of ERBB2 ("on-target-off-tumor"). Additional challenge remains on-target-off tumor attack by the CAR cells, for example cardiotoxicity, neurotoxicity, and anaphylaxis.

Such challenges to adoptive cell therapy treatment remain and accordingly, novel therapeutic strategies for treating solid tumors are unmet medical needs.

### SUMMARY

This summary is provided to introduce a selection of concepts in a simplified form that are further described below in the Detailed Description. This summary is not intended to identify key features of the claimed subject matter, nor is it intended to be used as an aid in determining the scope of the claimed subject matter.

In various aspects, the present disclosure provides a transpapillary method of adoptive cell therapy for the treatment of a subject having or at risk of having a breast disorder comprising administering cells into a breast duct.

In various aspects, the present disclosure provides transpapillary methods of adoptive cell therapy for treatment of a subject having or at risk of having a breast disorder comprising administering cells into a breast duct of the subject. The cells can be T-cells, NK cells, CTLs, TILs, monocytes, granulocytes, or progenitors thereof.

In one aspect, the cells comprise modified cells expressing one or more recombinant receptors that bind a target antigen on a breast cell of the subject. The recombinant receptors can be chimeric antigen receptors (CARs) or engineered or disease-specific T-cell receptors (TCRs). The engineered receptors in some embodiments are transgenic TCRs. In some embodiments, disease-specific TCRs are tumor-specific TCRs.

In some embodiments, the modified cells express two or more CARs. In other embodiments, the recombinant receptors, such as the CARs, can be independently monospecific, bispecific or multispecific. In yet other embodiments, the recombinant receptor is constitutively, transiently or switchably expressed, or conditionally active.

In another aspect, the disclosure provides that the modified cells comprise a safety switch that is capable of switching off the expression of the modified cells expressing a recombinant receptor. In some embodiments, the safety switch is selected from the group consisting of death gene switches, FITC-based switches, and PNE-based switches. In at least one embodiment, the safety switch is a death gene switch. In other embodiments, the death gene switch is a HSV-tk, an iCaspase9 or a FADD.

In one aspect, the present disclosure provides that target antigen to which an Ag-binding domain of a recombinant receptor binds is a tumor specific antigen, a tumor associated antigen, a multi-lineage tumor associated antigen, an oncofetal antigen, a neoantigen, or an immunosuppressive antigen. In some embodiments, the target antigen is selected from the group consisting of transformation-related molecules such as MUCs such as MUC1, c-met, cytokeratins such as CK5, CK6, CK14, CK7, CK8, CK14, CK17, CK18, CK19, p53, glycosides, Tn, TF, and sialyl Tn (STn), Lewis x, Lewis a, Lewis y, and gangliosides such as GM3, GD3, 9-0-acetyl GD3, 9-0-acetyl GT3, and N-glycoly-GM3, Folate Receptor alpha, ROR1, neoantigens, tumor-specific antigens and oncofetal antigens, tumor associated antigens such as carcinoembryonic antigen (CEA), L1 cell adhesion molecule (LI CAM), CAFs-related proteins such as fibroblast activation protein (FAP), FAP-α, FSP-1/S100A4, and PDGFR-β, diganglioside GD2, mesothelin, IL-13 receptor IL13R, IL-13 receptor α, ephrinB2, IGFR1, ELIGHT, WT1, TAG-72, Ep-CAM, LFA-1, EGFR, estrogen receptor (ER), progesterone receptor, MAGE1, MAGE-3, MAGE-A3/6, MAGE-A family members such as MAGE-A1, MAGE-A2, MAGE-A3, MAGE-A4, MAGE-A6, MAGE-A12, MAGE-A9, MAGE-A11, MAGE-C1, and MAGE-C2, RAGE, BCR-ABL, protein tyrosine kinases such as PRL-2 and PRL3, tumor associated glycoproteins such as TAG-72, CA 19-9, CA 27.29, CA 72-4, CA 50, PD-1, CTLA-4, CD47, receptor tyrosine kinases such as H4-RET, Ki-67, cyclin D1, cyclin A, cyclin E, p16, p21, p27, p53, Bcl-2, Bax, survivin, c-myc, Rb, VEGF, HPR1, HER1, HER2, HER3, HER4, CD10, SPARC, COX-2, basal cytokeratins, CK5/6, CK14, and CK17, epidermal growth factor receptor, c-kit, c-erbB-2, IL-10, TGF-beta, CCL17, CCL22, and CCL24 stroma released factors such as EGF, HGF, MCP-1, CSF-1, VEGF, cytokines such as IL1, IL-8, TNF-alpha, enzymes such as MM2, MMP7, MMP8, MMP9, MMP12, MMP13, and COX2. In some embodiments, the recombinant receptor is a HER2-CAR, FR-α-CAR, or a FAP-CAR.

In one aspect, the recombinant receptor comprises a primary signaling molecule selected from the group consisting of TCR zeta, FcR gamma, FcR beta, CD3 gamma, CD3 delta, CD3 epsilon, CD4, CDS, CD16, CD22, CD25, CD79a, CD79b, and CD66d. In another aspect, the recombinant receptor comprises one or more co-stimulatory molecules. In some embodiments, one or more co-stimulatory molecule is independently selected from the group consisting of MHC class I molecule, BTLA and a Toll ligand receptor, immunoglobulin superfamily (IgSF) such as CD28, 137 receptor family members (B7-H2/B7RP-1/LICOS/GL50, B7-DC/PD-L2, B7-H3), CD226, TIM, CD2/SLAM, BTN, LAIR, tumor necrosis factor receptor superfamily (TNFRSF) such as OX40, CD27, CD30, DR3, GITR, and HVEM, CD2 and SLAM on T-cells, ICAM-1, LFA-1 (CD11a/CD18), adhesion molecules (CD54, CD58, CD70), ICOS, CD40, CD40L, 4-1BB (CD137), CD70, CD80, CD86, DAP10, and other orphan receptor families such as LAG3 (CD223) and CD160. In at least one embodiment, the recombinant receptor comprises CD28 and 4-1BB as co-stimulatory molecules.

In one aspect, the present disclosure provides that the modified cell is a T-cell, an NK cell, a CTL, a monocyte, a granulocyte, or progenitors thereof.

In another aspect, the present disclosure provides that the modified cells further comprise a dye or a contrasting agent selected from the groups consisting of gadolinium chelates, superparamagnetic iron oxide nanoparticles (SPION), ¹⁹F perfluorocarbon nanoparticles, and other magnetic reporter genes, such as metalloprotein-based MRI probes. Dye or contrast agent enables the monitoring of the rate of migration and tumor infiltration of the administered cells and appearance of the cells in blood and other tissues.

In yet another aspect, the present disclosure provides that the cells are formulated in a composition suitable for transpapillary administration into a breast duct of a subject. Such a composition further comprises a pharmaceutically acceptable carrier, buffer, an excipient or a combination thereof. In at least one embodiment, the carrier is lactated Ringers Solution. In some embodiments, the composition further comprises a gelling agent.

In still another aspect, the present disclosure provides that the breast disorder is benign breast disease, breast cancer, Paget's disease of the nipple, or phyllodes tumor. In some embodiments, the breast disorder is hyperplasia, atypia, ductal hyperplasia, lobular hyperplasia, atypical ductal hyperplasia (ADH), or atypical lobular hyperplasia (ALH).

In other embodiments, the breast disorder is a breast cancer selected from the group consisting of ductal carcinoma in situ (DCIS), lobular carcinoma in situ (LCIS), invasive (or infiltrating) lobular carcinoma (ILC), invasive (or infiltrating) ductal carcinoma (IDC), microinvasive breast carcinoma (MIC), inflammatory breast cancer, ER-positive (ER+) breast cancer, progesterone receptor positive (PR+) breast cancer, ER+/PR+ breast cancer, ER-negative (ER-) breast cancer, HER2+ breast cancer, triple negative breast cancer (i.e., ER-/PR-/Her2-breast cancer; "TNBC"), adenoid cystic (adenocystic) carcinoma, low-grade adenosquamatous carcinoma, medullary carcinoma, mucinous (or colloid) carcinoma, papillary carcinoma, tubular carcinoma, metaplastic carcinoma, and micropapillary carcinoma.

In yet other embodiments, the breast cancer is a pre-cancer, an early stage cancer, a non-metastatic cancer, a pre-metastatic cancer, a locally advanced cancer, a metastatic cancer or a recurrent cancer.

In an aspect, the present disclosure provides that the cells are administered in a single dose or multiple doses. Where the cells are administered in multiple doses, the multiple doses comprise a first dose and one or more subsequent doses. The present disclosure further provides that one or more dose is administered in a split unit dose.

In another aspect, the subject is administered a unit dose of cells ranging from 1×10³ to 1×10⁹ modified cells/kg body weight, from 1×10³ to 5×10⁸ modified cells/kg body weight, from 0.5×10³ to 1×10⁷ modified cells/kg body weight, from 1×10⁴ to 0.5×10⁶ modified cells/kg body weight, from 0.5×10⁴ to 1×10⁶ modified cells/kg body weight, from 1×10⁵ to 0.5×10⁶ modified cells/kg body weight. In some embodiments, the first dose is a low dose, such as less than 1×10³ cells/kg, less than 0.5×10⁴ cells/kg, less than 1×10⁴ cells/kg, less than 0.5×10⁵ cells/kg, less than 1×10⁵ cells/kg, less than 0.5×10⁶ cells/kg, or less than 1×10⁶ cells/kg.

In other embodiments, the first dose is a high dose, such as greater than 0.5×10⁵ cells/kg, greater than 1×10⁵ cells/kg, greater than 0.5×10⁶ cells/kg, greater than 1×10⁶ cells/kg, greater than 0.5×10⁷ cells/kg, greater than 1×10⁷ cells/kg, greater than 0.5×10⁸ cells/kg, greater than 1×10⁸ cells/kg, or greater than 5×10⁸ cells/kg.

In yet other embodiments, a subsequent dose is administered between 7 and 28 days, each inclusive, after the initiation of the first dose. In further embodiments, a subsequent dose is same as the first dose, lower than the first dose or higher than the first dose.

In an aspect, transpapillary adoptive therapy can be primary therapy, neoadjuvant therapy, or adjuvant therapy. In some embodiments, the cells are administered as primary therapy, neoadjuvant therapy, or adjuvant therapy.

In an aspect, the present disclosure provides that the administration of cells reduces disease burden of the breast disorder in the subject. In some embodiments, the administration of cells reduces tumor burden. In other embodiments, the administration of modified cells comprising a recombinant receptor reduces tumor burden in a subject. In yet other embodiments, administration of cells reduces a risk of a CRS-related, a MAS-related, a TLS-related, a neurotoxicity-related or a host immune response-related outcome. In still other embodiments, administration of modified cells reduces a risk of a CRS-related, a MAS-related, a TLS-related, a neurotoxicity-related or a host immune response-related outcome. The administration of cells such as modified cells reduces circulating or breast tissue levels of cytokines such as IFNγ, TNFα, IL-2, GM-CSF, IL-1beta, IL-6, IL-7, IL-8, IL-10, IL-12, Flt-3, fractalkine, MIP1, sIL-2Rα, and IL-5.

The present disclosure further provides that the subject is preconditioned with a lymphodepleting agent or a chemotherapeutic agent prior to administration of the cells. In some embodiments, the lymphodepleting agent or a chemotherapeutic agent is selected from the group consisting of cyclophosphamide, cyclosporine, fludarabine, bendamustine, lenalidomide, pomalidomide, gemcitabine, BTK inhibitors such as ibrutinib, oncolytic adenovirus, and combinations thereof.

In one aspect, the method comprises administration of an additional therapeutic agent or therapy to the subject. In some embodiments, the additional therapeutic agent is selected from the group consisting of asparaginase, busulfan, carboplatin, cisplatin, daunorubicin, doxorubicin, fluorouracil, gemcitabine, hydroxyurea, methotrexate, paclitaxel, rituximab, vinblastine, vincristine, oncolytic viruses such as oncolytic adenovirus, anti-estrogens such as tamoxifen, N-methyl-endoxifen, norendoxifen, endoxifen, raloxifen, fulvestrant and/or aromatase inhibitors such as anastrozole, letrozole, and exemestane.

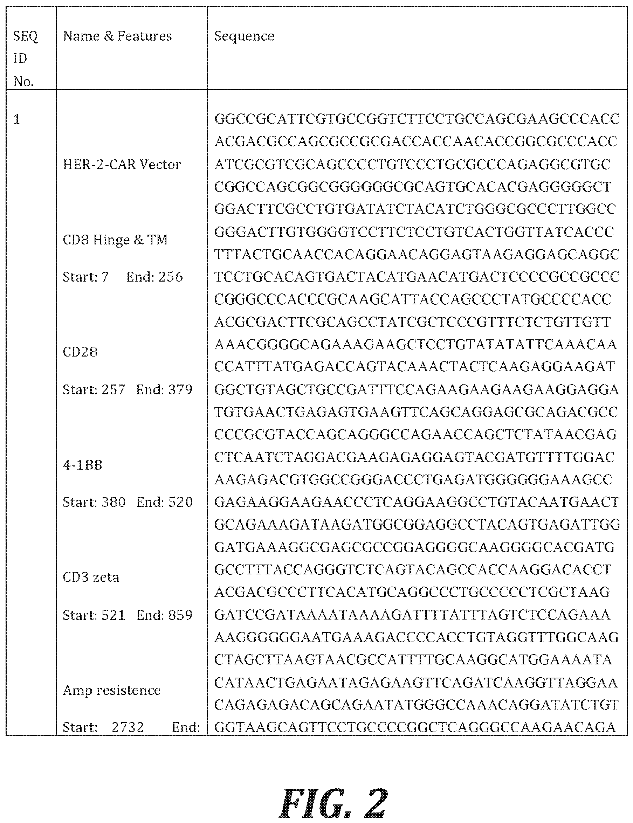

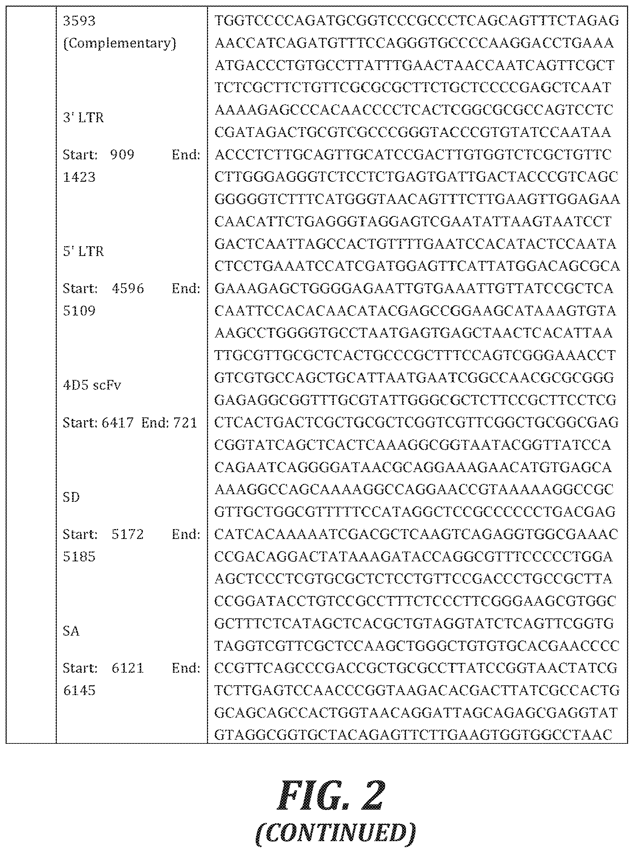

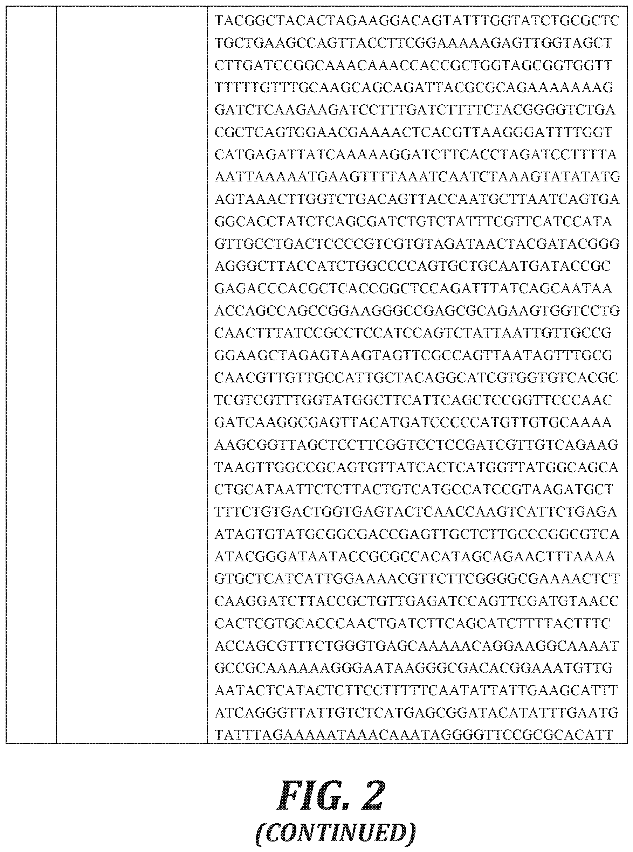

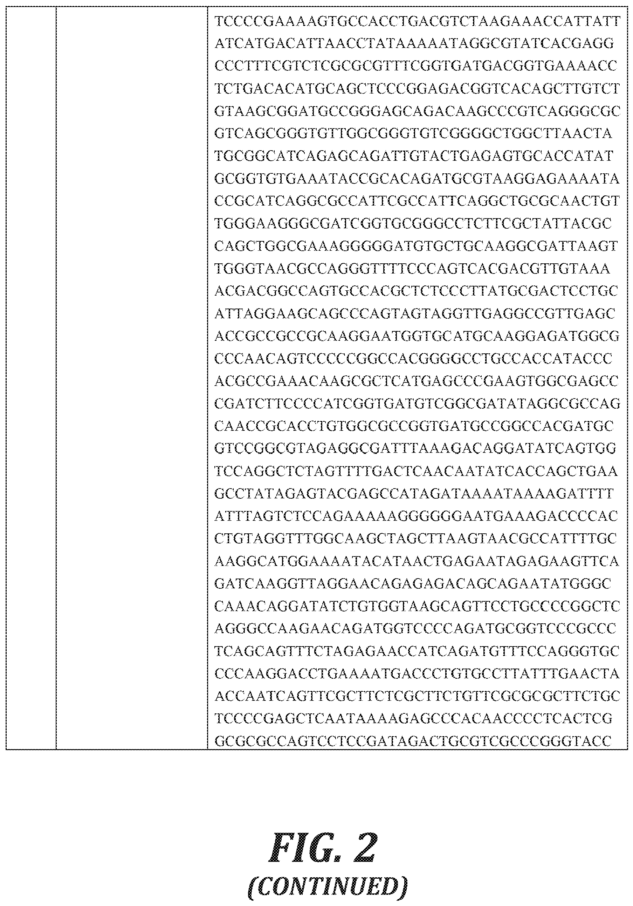

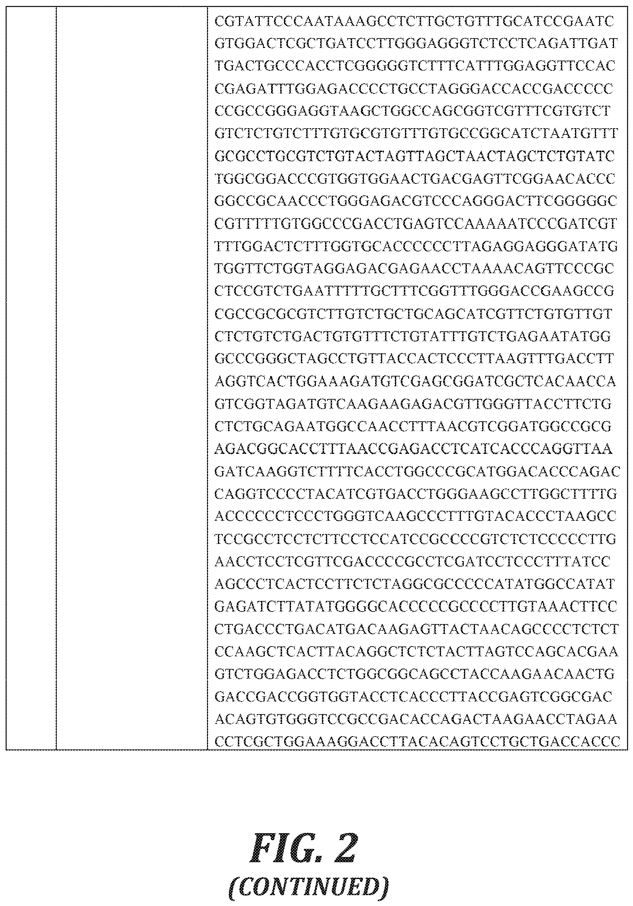

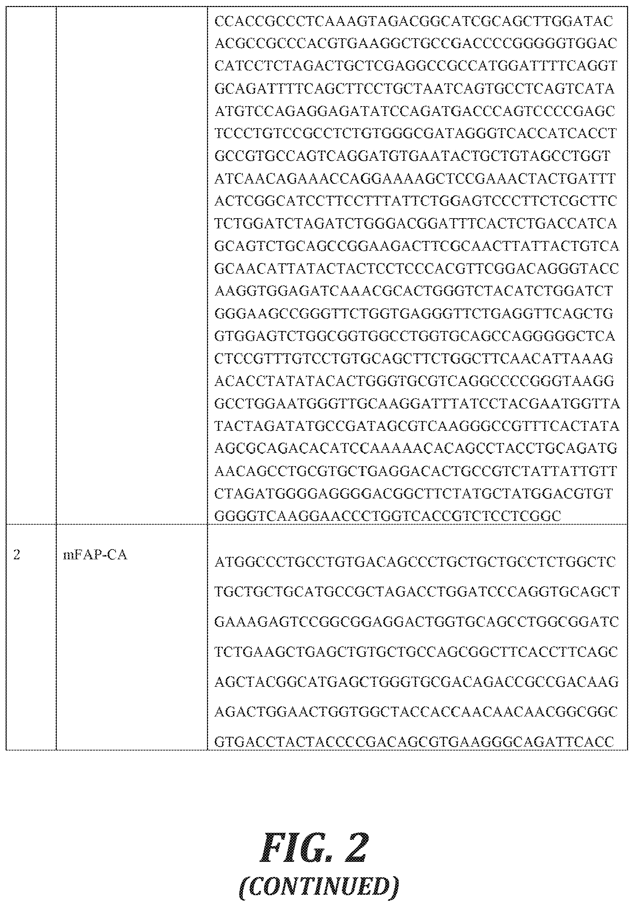

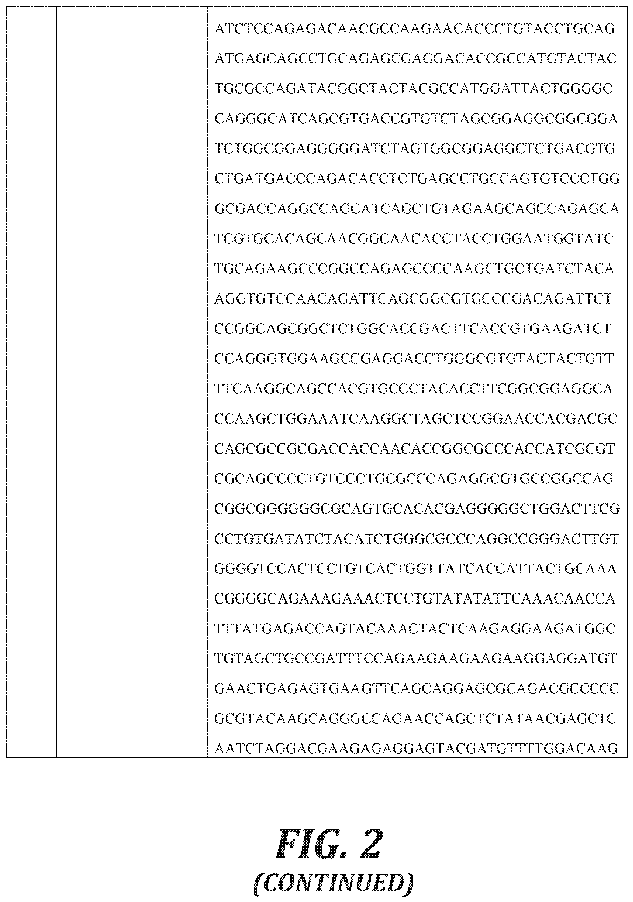

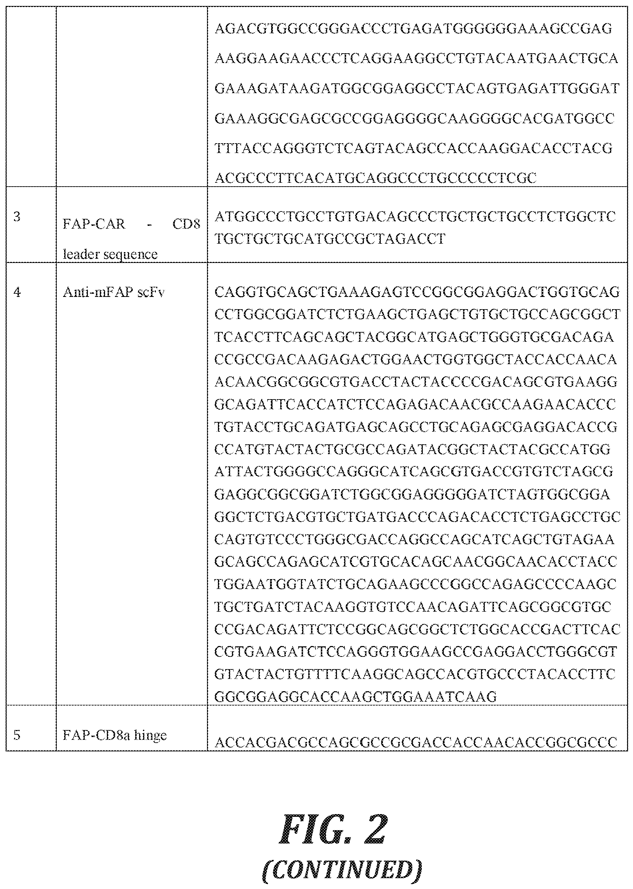

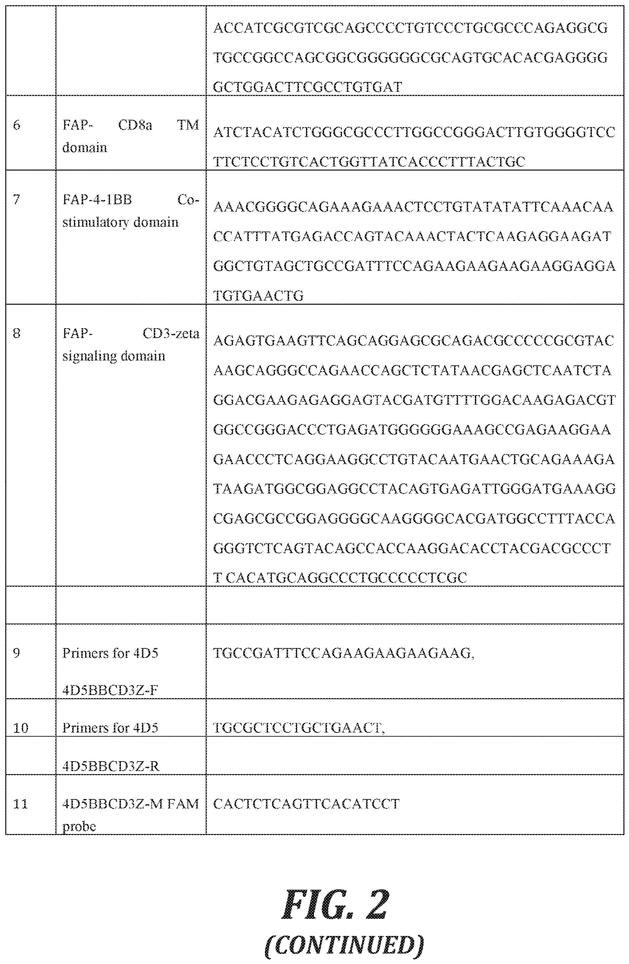

Provided herein are transpapillary methods of adoptive cell therapy for treatment of a subject having or at risk of having a breast disorder comprising administering modified cells expressing a HER2-CAR, a FAP-CAR, or a FR-α into a breast duct of the subject. In some embodiments, the HER2-CAR has at least 80%, at least 81%, at least 82%, at least 83%, at least 84%, at least 85%, at least 86%, at least 87%, at least 88%, at least 89%, at least 90%, at least 91%, at least 92%, at least 93%, at least 93%, at least 94%, at least 95%, at least 96%, at least 97%, at least 98%, at least 99%, and 100% homology to SEQ ID NO:1 disclosed in FIGURE 2 or a variant or functional portion thereof.

In other embodiments, the FAP-CAR has at least 80%, at least 81%, at least 82%, at least 83%, at least 84%, at least 85%, at least 86%, at least 87%, at least 88%, at least 89%, at least 90%, at least 91%, at least 92%, at least 93%, at least 93%, at least 94%, at least 95%, at least 96%, at least 97%, at least 98%, at least 99%, and 100% homology to SEQ ID NO:2 disclosed in FIGURE 2 or a variant or functional portion thereof.

In another aspect, the present disclosure provides an article of manufacture, comprising one or more containers, packaging material, a label or package insert, and optionally, a device. In some embodiments, the device is a needle and syringe, a cannula, a catheter, a microcatheter, an osmotic pump, or an encapsulation device.

### DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:
The foregoing aspects and many of the attendant advantages of this invention will become more readily appreciated as the same become better understood by reference to the following detailed description, when taken in conjunction with the accompanying drawings, wherein:

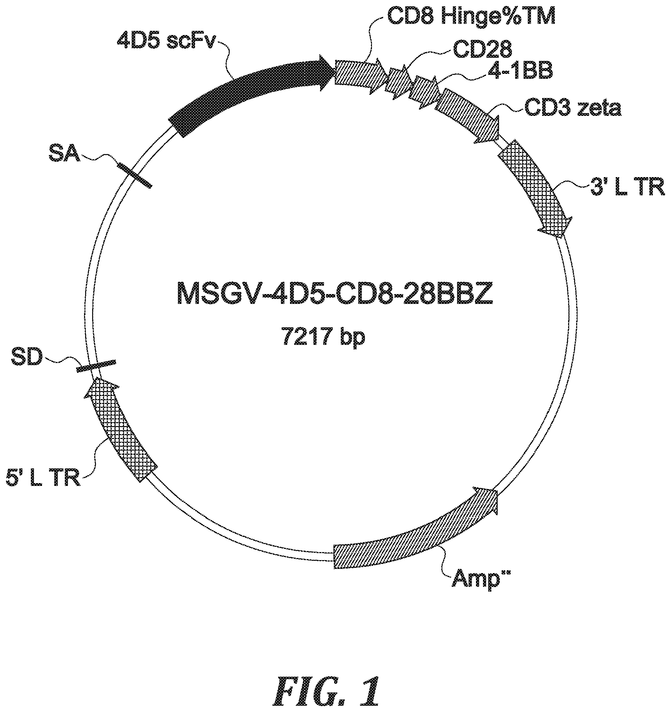

FIGURE 1 schematically illustrates ErbB2 (HER2) CAR vector (gamma-retroviral vector MSGV1-4D5-CD8-28BBZ) used to transduce peripheral blood lymphocytes (Morgan et al., Molecular Therapy, 2010, 18(4), 843-851).
FIGURE 2 is a table with sequences for HER2-CAR vector and FAP-CAR domains.

### DETAILED DESCRIPTION

### Methods of Treatment

Provided herein are novel methods of treating breast disorders in a subject using adoptive cell therapy. The methods comprise administering adoptive cell therapy to a breast milk duct of a subject having or at risk of having a breast disorder. Cells used in adoptive cell therapy includes, but is not limited to, genetically modified cells such as T lymphocytes (T-cells), natural killer (NK) cells or cytotoxic T lymphocytes (CTLs), monocytes, granulocytes, normal immune cells, and progenitors thereof. The methods for treatment of breast disorders provided herein further comprise administration of additional therapeutic agents or therapies.

A "modified cell" as used herein is a genetically engineered cell that expresses at least one recombinant receptor designed to recognize and/or specifically bind to a "target antigen" molecule associated with a disease or a condition and result in a response, such as an immune response against such molecules upon binding to target antigen molecule. The recombinant receptors include, for example, "chimeric antigen receptors" (CARs) and other engineered and disease specific antigen receptors such as tumor-specific and transgenic T-cell receptors (TCRs).

Although subjects have been treated with genetically modified cells via intravenous, intratumoral, and parenteral injections for the treatment of solid tumors in various clinical trials to date, the treatment with such compositions remains challenging due to limited ability of the cells to successfully migrate and infiltrate solid tumors and rapidly make contact with target tumor cells (Guo et al., J. Immunol. Res. 2016, Article ID. 3850839). This can be due to the barrier functions performed by tissues, low persistence of the modified cells after administration, and the hostile tumor microenvironment, both physical and metabolic (Newick et al., Molecular Therapy - Oncolytics. (2016) 3, 16006). As a result, low antitumor cytotoxicity of the cells is often observed. Although inclusion of co-stimulatory molecules in the modified cells can mitigate some of the limitations, better methods of treatment of breast disorders would be more beneficial and remain unmet need.

Disclosed herein are novel methods of adoptive cell therapy wherein cells or compositions are administered transpapillarily to at least one breast milk duct ("breast duct" or "duct" hereinafter) of a subject. In an aspect, the route of administration of adoptive cell therapy is transpapillary.

As used herein, the terms "transpapillary" and "transpapillarily" refer to a method of treatment wherein cells for adoptive cell therapy are delivered into the lumen of at least one breast milk duct of a subject through the milk duct opening (ductal orifice) in the nipple on the mammary papilla(e) to reach the inner depths of the breast.

In one aspect, transpapillary administration refers to the application of the adoptive cell therapy compositions of the present disclosure to the nipple of a mammary papilla, wherefrom the compositions are delivered to at least one breast milk duct through a ductal orifice in the nipple. Breast ducts are uniquely suited for receiving prophylactic and therapeutic agents via ductal orifices.

In another aspect, transpapillary administration refers to the intraductal delivery of cells for adoptive cell therapy directly into the lumen of a breast milk duct via a ductal orifice in the nipple of a mammary papilla. For the purpose of the present invention, the terms "intraductal" and "intraductally" refers to transpapillary delivery wherein the compositions are not applied to nipples. It will be appreciated by a person of skill in the art that transpapillary methods as disclosed herein comprise delivery of the cells for adoptive cell therapy through a natural orifice of the breast milk duct in a subject's breast, and that intraductal delivery for the purpose of the present invention is a form of transpapillary delivery. An advantageous aspect of the present invention is that transpapillary delivery typically does not involve deliberate breach of subject's skin or ti ssue or cell layer.

Breast disorders, such as breast cancers, typically originate in a milk duct of an individual (Wellings SR. Pathol. Res. Prac. 1980; 166:515-535; Love and Barsky. Cancer. 2004, 101(9):1947-1957). Thus, localized delivery of the cells, for example, modified cells expressing recombinant receptors such as CARs and engineered TCRs, close to the affected site within the breast milk ducts (breast ducts) is highly desirable. Transpapillary administration of such cells or compositions comprising such cells providing a local, effective, easy-to-administer therapy would obviate the side effects of systemic treatment by reducing systemic exposure to the compositions comprising modified cells. This would have the added benefit of reducing the possibility/risk of on-target-off-tumor effect. Local delivery to the breast duct by intraductal administration would also allow greater local expansion of the cells, reduced migration time, and faster access to the affected tissues in shorter period and thereby, improving cytotoxic activity and efficacy.

As used herein, "breast disorder" means any aberration or a constellation of aberrations in the breast. Such aberration may be proliferative, non-proliferative, benign or malignant. Breast disorders include benign lesions of the breast (e.g., hyperplasia), Paget's disease of the nipple, phyllodes tumor, and breast cancer. Benign breast lesions include, but are not limited to, hyperplasia, atypia, ductal hyperplasia, lobular hyperplasia, atypical ductal hyperplasia (ADH), and atypical lobular hyperplasia (ALH). While not cancerous, ADH and ALH may be indicative of a predisposition for breast cancer

As used herein, "breast cancer" means any malignant tumor of breast cells. Breast cancer may be at any stage of breast cancer, including stages of a pre-cancer, an early stage cancer, a non-metastatic cancer, a pre-metastatic cancer, a locally advanced cancer, and a metastatic cancer. There are several types of breast cancer. Exemplary breast cancers include, but are not limited to, ductal carcinoma in situ (DCIS), lobular carcinoma in situ (LCIS), invasive (or infiltrating) lobular carcinoma (ILC), invasive (or infiltrating) ductal carcinoma (IDC), microinvasive breast carcinoma (MIC), inflammatory breast cancer, ER-positive (ER+) breast cancer, progesterone receptor positive (PR+) breast cancer, ER+/PR+ breast cancer, ER-negative (ER-) breast cancer, HER2+ breast cancer, triple negative breast cancer (i.e., ER-/PR-/Her2-breast cancer; "TNBC"), adenoid cystic (adenocystic) carcinoma, low-grade adenosquamatous carcinoma, medullary carcinoma, mucinous (or colloid) carcinoma, papillary carcinoma, tubular carcinoma, metaplastic carcinoma, or micropapillary carcinoma. A single breast cancer tumor can be any combination or a mixture of these types or be a mixture of invasive and in situ cancer.

DCIS is the most common non-invasive breast cancer. It involves the cell(s) lining the breast ducts. In DCIS, the cells have not spread beyond the walls of the duct into the surrounding breast tissue. About 1 in 5 new breast cancer cases will be DCIS. Several biomarkers are associated with DCIS. Exemplary biomarkers include, without limitation, estrogen receptor, progesterone receptor, androgen receptor, Ki-67, cyclin D1, cyclin A, cyclin E, p16, p21, p27, p53, Bcl-2, Bax, survivin, c-myc, Rb, VEGF, HPR1, HER1, HER2, HER3, HER4, CD10, SPARC, COX-2, basal cytokeratins, CK5/6, CK14, and CK17, epidermal growth factor receptor, Tn, ER+, and c-kit. DCIS is said to be a non-obligate precursor to IDC. Involvement of stromal cells, such as cancer-associated fibroblasts (CAFs), which secrete certain HGF and fibroblast activating protein (FAP) aid in DCIS cells become invasive and develop into IDC.

LCIS is a pre-cancerous neoplasia. It may be indicative of a predisposition for invasive cancer. LCIS only accounts for about 15% of the in situ (ductal or lobular) breast cancers. LCIS biomarkers include, but are not limited to, E-cadherin, ER, PgR, c-erbB-2, p53 and Ki-67.

IDC is the most invasive breast cancer. As the name applies, it is a carcinoma that begins in the breast ducts and then invades the surrounding fatty tissue. About 8 to 10 invasive breast cancers are infiltrating ductal carcinomas. IDC is often treated by surgery to excise the cancerous tissue, and radiation therapy. In addition, chemotherapy combined with immunotherapy (e.g., tamoxifen and tratuzumab) is often used to treat IDC. If the tumor is larger than 4 cm, then a radical mastectomy may be performed. Biomarkers for IDC include but are not limited to carbohydrate antigens such as Tn, Tf, sialyl-Tn, Lewis x, Lewis a, Lewis y, and gangliosides such as GM3, GD3, 9-0-acetyl GD3, 9-0-acetyl GT3, N-glycoly-GM3.

ILC is a cancer that develops in the lobules of the breast and has invaded the surrounding tissue. About 1 in 10 invasive breast cancer is an ILC. ILC is treated by surgery to excise the cancerous tissue, and radiation therapy. In addition, chemotherapy and immunotherapy combination (e.g., tamoxifen and tratuzumab) is often used as an adjuvant therapy to treat ILC. Like IDC which is invasive with the aid of growth factors and cytokines released by cancer-associated fibroblasts (CAPs), ILC too is aided by CAFs-related proteins FAP-α, FSP-1/S100A4, and PDGFR-β.

Inflammatory breast cancer accounts for about 1% to 3% of all breast cancers. In inflammatory breast cancer, cancer cells block lymph vessels in the skin resulting in the breast turning red and feeling warm. The affected breast may become larger or firmer, tender, or itchy. Inflammatory breast cancer is treated with chemotherapy, immunotherapy, radiation therapy, and in some cases, surgery.

Estrogen Receptor positive (ER+) breast cancer is characterized by the presence of estrogen receptors on the surface of the cancerous cells. Growth of ER+ cancer cells is associated with the availability of estrogen (hormone-dependent or hormone sensitive breast cancer). Approximately, 80% of all breast cancers are ER+ breast cancers. Treatment options for ER+ breast cancer include chemotherapeutic agents that block estrogen (e.g., tamoxifen).

Triple negative breast cancer is characterized by the absence of estrogen receptor, progesterone receptor, and HER2 receptor and occurs in about 10% - 20% of diagnosed breast cancers. TNBC can be very aggressive and a subject has a risk of reoccurrence. Treatment options typically includes neoadjuvant chemotherapy (but not an anti-estrogen such as tamoxifen or anti-HER2 such as trastuzumab) followed by surgery such as lumpectomy. Further, TNBC is a highly diverse group of cancers and has been subtyped into at least 6 TNBC subtypes displaying unique gene expression and ontologies, including 2 basal-like (BL1 and BL2), an immunomodulatory (IM), a mesenchymal (M), a mesenchymal stem-like (MSL), and a luminal androgen receptor (LAR) subtype (Lehrmann et al., J. Clin. invest, 2011, 121(7), 2750-2767) incorporated by reference herein in its entirety. Mutations and markers have been described that are can target specifically the TNBC subtypes (*Id*). Additional biomarkers for TNBC include, but are not limited to, Epidermal growth factor receptor, folate receptor-α, vascular endothelial growth factor, c-Myc, C-kit and basal cytokeratins, Poly(ADP-ribose) polymerase-1, p53, tyrosinase kinases, m-TOR, heat and shock proteins and TOP-2A. Further, stromal cells (cancer-associated fibroblasts (CAFs) and immune cells such as tumor associated macrophages (TAMs) and Tumor associated neutrophils (TANs)) surrounding tumors play an important role in creating barriers to therapeutic drug penetration by through increased deposition of extracellular matrix and release of various profibrotic growth factors such as TGF beta, bFGF, and other factors that impact cancer cell proliferation, invasion and metastasis (for example by promoting epithelial-to-mesenchymal transition) such as fibroblast activating protein (FAP), EGF, HGF, MCP-1, CSF-1, VEGF, cytokines such as IL1, IL-8, TNF-alpha, enzymes such as MM2, MMP7, MMP8, MMP9, MMP12, MMP13, and COX2. TAMs further suppress anti-tumor immune response by secreting cytokines and chemokines (for example IL-10, TGF-beta, CCL17, CCL22, and CCL24) favoring the recruitment of T-reg cells and generation of immune suppressive microenvironment.

In some embodiments, the subject's breast disorder is breast cancer, Paget's disease of the nipple, or phyllodes tumor. In yet other embodiments, the breast cancer is a pre-cancer, an early stage cancer, a non-metastatic cancer, a pre-metastatic cancer, a locally advanced cancer, or a metastatic cancer.

In other embodiments, the subject has breast cancer selected from the group consisting of ductal carcinoma in situ (DCIS), lobular carcinoma in situ (LCIS), invasive (or infiltrating) lobular carcinoma (ILC), invasive (or infiltrating) ductal carcinoma (IDC), microinvasive breast carcinoma (MIC), inflammatory breast cancer, ER-positive (ER+) breast cancer, progesterone receptor positive (PR+) breast cancer, ER+/PR+ breast cancer, ER-negative (ER-) breast cancer, HER2+ breast cancer, triple negative breast cancer (i.e., ER-/PR-/Her2- breast cancer; "TNBC"), adenoid cystic (adenocystic) carcinoma, low-grade adenosquamatous carcinoma, medullary carcinoma, mucinous (or colloid) carcinoma, papillary carcinoma, tubular carcinoma, metaplastic carcinoma, and micropapillary carcinoma.

In some embodiments, the subject has persistent or relapsed disease, i.e., following treatment with another therapeutic intervention, including chemotherapy (such as gemcitabine, tamoxifen, trastuzumab, etc.) or radiation. In other embodiments, the subject has become resistant to another therapeutic drug. As used herein, the term "resistance" refers to two classes of resistance: (a) de novo resistance, i.e., non responsiveness to therapy or therapeutic from the beginning of the treatment, and (b) acquired resistance, i.e., non-responsiveness to therapy or therapeutic after initial responsiveness or therapeutic-dependent growth /stimulated growth while continuing to express receptors (or ligands as therapeutic-appropriate).

In some embodiments, the subject is tamoxifen resistant or tamoxifen refractory. As used herein, the term "tamoxifen refractory" refers to subjects that have been dosed daily with tamoxifen for at least 2 days and have a level of plasma endoxifen of less than 15 nM (e.g., less than 20 nM, less than 25 nM, or less than 30 nM). The acquired resistance to tainoxifen may develop as early as 3 m to 1 year to as late as 5 to 10 years. In one aspect, transpapillary methods of treatment are particularly desirable for the treatment of pre-cancers, early stage cancers, non-metastatic cancers, pre-metastatic cancers, and locally advanced cancers. In another aspect, transpapillary methods of treatment are also suitable for the treatment of metastatic cancers.

Accordingly, disclosed herein for the first time are transpapillary methods of adoptive cell therapy for the treatment of a subject having or at risk of having a breast disorder. Cells or compositions disclosed herein are administered to a breast milk duct lumen of the subject. In a novel aspect, transpapillary methods of treatment disclosed herein are non-invasive or minimally-invasive, and typically do not involve breaking skin or tissue barrier. Instead, the cells are administered to a subject via the subject's own natural ductal orifice in the nipple of a mammary papilla, wherein breast carcinomas such as DCIS typically originate.

In another novel aspect, this invention encompasses delivering cells or compositions disclosed herein to the apical surface of a tumor in a breast milk duct. In the breast, the breast duct has a bilayer of epithelial cells; the inner layer is made from polarized luminal cells that are surrounded by an outer myoepithelial cell layer, which contacts the basement membrane. The apical surface of the breast epithelial cells faces the central lumen of the duct, into which milk is secreted during pregnancy. The basolateral domain makes contact with neighboring luminal cells as well as myoepithelial cells and the basement membrane (Chatterjee and McCaffrey. Breast Cancer: Targets & Therapy. 2014:6 15-27). The cells administered into the lumen of a breast duct can migrate within the lumen (and/or microlumen) of a duct across the apical end into the breast or tumor tissue. Accordingly, in some embodiments, modified cells approach and conduct an apical attack on the tumor rather than basolateral attack. Apical attack by modified cells as disclosed herein is desirable for treatment of pre-metastatic cancers in particular. Current methods of treatment typically involve the administration of adoptive cell therapy, such as the CAR-T cells, parenterally by injections and infusions, generally intravenous or intratumoral. Additional methods of delivery known in the art include subcutaneous, intranasal, intraperitoneal, intrapulmonary, intraarterial, intramuscular, and intraperitoneal, etc. (see, e.g., US 2016/0045551; U.S. Patent No 9,365,641, US 2016/0206656). In each of these cases, the transferred cells are required to migrate across long distances and survive for periods long enough to contact affected tissues. The approach in these methods is basolateral attack of the tumor

Disclosed herein are transpapillary methods of administering cells or composition comprising modified cells to a breast milk duct of an subject having or at risk of having a breast disorder. In some embodiments, the cells or compositions are administered into one breast milk duct. In other embodiments, the cells or compositions disclosed herein are administered into 2 to 5 breast ducts, into 4 to 8 breast ducts, or into 7 to 11 breast ducts.

Cells or compositions comprising cells may be delivered transpapillarily into a breast duct by any of the methods known in the art. These include, but are not limited to, injections using syringe/needle (Krause et al., J. Vis. Exp. 2013; (80): 50692); cannula(e), catheters, probes, as well as those disclosed in U.S. Patent Nos. 6,413,228; 6,689,070; 6,638,727, patent application PCT/US2015/010808), time-release capsules and encapsulation devices etc.

As a non-limiting example, in some embodiments, cells or compositions disclosed herein may be administered to a breast milk duct of the subject comprising (a) contacting a composition comprising modified cells, contained within a treatment chamber of a device with a nipple of a breast; and (b) applying positive pressure on the composition. In some embodiments, the composition is forced into the breast duct due to the positive pressure. Preferably, the composition is forced into one or more breast ducts. In other embodiments, the composition is forced into 2 to 5 breast ducts, into 4 to 8 breast ducts, or into 7 to 11 breast ducts.

For example, U.S Patent No. 6,413,228 discloses a ductal access device that is capable of collecting ductal fluid and infusing the ductal with wash fluid. Such a device can be adapted or configured appropriately for the purpose of this disclosure for the transpapillary delivery of cells or compositions of the present invention.

In some embodiments, the device is a cannula.

As an alternate method of intraductal administration, a small pump may be installed in the duct or at the surface of the nipple with access to the duct for slow continuous administration of modified cells to the ductal region, e.g., a pump may be installed in the lactiferous sinus for administering the modified cells therein and causing a diffusion of the cells to the rest of the duct or the pump may be installed on the nipple surface with access to the duct. A pump installed at the nipple surface can be shaped e.g., like a tack (or a thimble-shaped portion having a top or tack portion and the rest on the nipple surface with a portion extending into a duct requiring treatment or having a risk of requiring treatment. The pump mechanism can comprise e.g. a Duros^{®} osmostic (micro)pump (Viadur), manufactured by Alza Corp acquired by Johnson & Johnson, IntelliDrug, Alzet^{®} (Durect Corp.), Ivomec SR^{®} bolus etc. (Herrlich et al., Advanced Drug Delivery Reviews, 2012, pages 1617-1626).

Osmotic pumps may also be assembled or configured essentially as the pumps described in U.S. Patent No. 5,531,736, U.S. Pat. No. 5,279,608, U.S. Patent No. 5,562,654, U.S. Patent No. 5,827,538, U.S. Patent No. 5,798,119, U.S. Patent No. 5,795,591, U.S. Patent No. 4,552,561, or U.S. Patent No. 5,492,534, with appropriate modifications in size and volume for administration to the duct of a breast, e.g. for placement into the duct (e.g. the lactiferous sinus) or for placement on the nipple surface. The tip (that accesses the duct) may be able to rotate in order to accommodate ducts of various positions on the nipple surface. A single tack-head pump can have one or more tips placed below the tack-head in order to access a particular duct or ducts, e.g. where two or more ducts in a breast need to be accessed. The pump so configured and loaded with appropriately formulated compositions comprising cells for intraductal administration, may administer modified cells as described, but may also contain and administer agents other than cells for an appropriate therapeutic purpose for treatment of a precancer or cancer condition in a breast duct. Conceivably the pump may be configured to administer to all the ducts located in the breast, with some size and volume alterations.

Cell encapsulation devices are another attractive method and include microencapsulation and macroencapsulation devices. Self-folding immune-protective cell encapsulation devices have been developed wherein cells are immune-isolated by surrounding them with a synthetic semipermeable nanoporous membrane that allows selective permeation of nutrients and therapeutics. Such encapsulation devices include pouches, fibers, beads, and any device made from semipermeable materials within which the cells are housed. Such devices may be configured for adoptive cell therapy. For example, devices can be prepared with biodegradable materials so as to release the cells within the duct.

Devices useful for the purpose of this invention may be implantable. For e.g., Steplian et al. have described biopolymer implants for delivery of adoptive cell therapy (Steplian et al., Nature Biotechnology, 2015, 33, 97-101). In an aspect, provided herein are devices that are implantable, for example, cannula(e), catheters, microcatheters, beads, encapsulation devices etc. Implantable devices for example may be configured to release cells and compositions of the present invention close to affected tissue and reduce their exposure to normal cells.

In another aspect, transpapillary delivery of the cells or compositions disclosed herein may be aided with iontophoresis which involves application of an electric current to the breast which aid the migration of the cells into and/or within a duct of the breast.

### Preconditioning

Preconditioning subjects with immunodepleting (e.g., lyinpliodepleting) therapies results in expansion of administered cells. For example, a T-cell can expand and acquire a memory phenotype that can improve the effects of adoptive cell therapy (ACT). Preconditioning with lymphodepleting agents such as cyclophosphamide, cyclosporine, fludarabine, bendamustine, lenalidomide, pomalidomide, gemcitabine, BTK inhibitors such as ibrutinib, oncolytic viruses such as oncolytic adenoviruses, including combinations thereof have been effective in improving the efficacy of transferred tumor infiltrating lymphocyte (TIL) cells in cell therapy, including to improve response and/or persistence of the transferred cells. Increased access to the homeostatic cytokines, such as IL-7 and IL-15, through elimination of the phenomenon known as "cytokine sinks", eradication of the suppressive influence of T-reg cells and enhancement of APC activation and availability appear to be the underlying mechanisms involved in this paradigm. See, e.g., Dudley et al., 2002 Science, 298, 850-54; Rosenberg et al., Clin Cancer Res 2011, 17(13):4550-4557. Likewise, in the context of CAR-T cells, several studies have incorporated lymphodepleting agents, most commonly cyclophosphamide, fludarabine, bendamustine, or combinations thereof, sometimes accompanied by low-dose irradiation. See Han et al., Journal of Hematology & Oncology 2013, 6:47; Kochenderfer et al., Blood 2012; 119: 2709-2720; Kalos et al., Set Transl Med 201 1, 3(95):95ra73; Clinical Trial Study Record Nos.: NCT02315612; NCT01822652. Such preconditioning can be carried out with the goal of reducing the risk of one or more of various outcomes that could dampen efficacy of the therapy. These include cytokine sink, by which T-cells, B cells, NK cells compete with TILs for homeostatic and activating cytokines, such as IL-2, IL-7, and/or IL-15; suppression of TILs by regulatory T cells, NK cells, or other cells of the immune system; impact of negative regulators in the tumor microenvironment. Muranski et al., Nat Clin Pract Oncol. 2006 December; 3(12): 668-681.

Thus, in some embodiments, the methods include administering a preconditioning agent, such as a lymphodepleting or chemotherapeutic agent, for example, cyclophosphamide, cyclosporine, fludarabine, bendamustine, lenalidomide, pomalidomide, gemcitabine, BTK inhibitors such as ibrutinib, oncolytic viruses such as oncolytic adenovirus, or combinations thereof, to a subject prior to the first or a subsequent dose. For example, the subject may be administered a preconditioning agent at least 2 days prior, such as at least 3, 4, 5, 6, or 7 days prior, to the first or a subsequent dose. In some embodiments, the subject is administered a preconditioning agent no more than 7 days prior, such as no more than 6, 5, 4, 3, or 2 days prior, to the first or subsequent dose.

Lymphodepleting agents described herein may be administered to a subject between 0.5 g/m² and 5 g/m², such as between 1 g/m² and 4 g/m², 1 g/m² and 3 g/m², or 2 g/m² and 4 g/m² of a lymphodepleting agent. In some aspects, the subject is administered 2 g/m² cyclophosphamide 2 g/m² of cyclophosphamide. In some embodiments, the subject is preconditioned with cyclophosphamide at a dose between 20 mg/kg and 100 mg/kg, such as between 40 mg/kg and 80 mg/kg. In some aspects, the subject is preconditioned with 60 mg/kg of cyclophosphamide. The amount of lymphodepleting agent to de administered will be determined by the attending physician.

In some embodiments, where the lymphodepleting agent comprises fludarabine, the subject is administered fludarabine at a dose between 1 g/m² and 100 g/m², such as between 10 g/m² and 75 g/m², 15 g/m² and 50 g/m², 20 g/m² and 30 g/m², or 24 g/m² and 26 g/m². In some instances, the subject is administered 25 g/m² of fludarabine. In some embodiments, the fludarabine can be administered in a single dose or can be administered in a plurality of doses, such as given daily, every other day or every three days. For example, in some instances, the agent, e.g., fludarabine, is administered between or between about 1 and 5 times, such as between or between about 3 and 5 times. In some embodiments, such plurality of doses is administered in the same day, such as 1 to 5 times or 3 to 5 times daily.

In some embodiments, lymphodepletion may be carried out with one or more lymphodepleting agent. For example, subjects receive cyclophosphamide intravenously over 1 hour on days -7 and -6 and fludarabine phosphate intravenous piggyback over 30 minutes on days -7 to -3. Then on day 0, subject is administered with the cells or compositions disclosed herein.

### Dosing

The timing and size of dosing of cells and compositions are generally designed to reduce risk of or minimize toxic outcomes and/or improve efficacy, such as providing faster and increased exposure of the subject to the cells, e.g., over time. The quantity and frequency of administration will be determined by such factors as the condition of the patient, age, weight, tumor size and stage, and severity of the subject's disease, although the appropriate dosage may be determined by attending physicians.

Optimal dosages and dosing regimen can be readily determined by a person of skill in the art of medicine by monitoring the patient for signs of disease and adjusting the treatment accordingly. Cells or compositions can be administered multiple times at these dosages. Accordingly, the methods can involve a single dose or multiple doses over a period of time or continue dose for, e.g., by infusion. In some embodiments, a dose can be a single unit dose. In other embodiments, a single dose can be a split unit dose. As used herein, the term "split unit dose" refers to a unit dose that is split so that it is administered over more than one time during a day, including over more than one day. As split unit dose for the purpose of this invention is considered a single, i.e., one unit dose. Exemplary methods of splitting a dose include administering 25% of the dose the first day and administering the remaining the next day. In another embodiment, the unit dose may be split into 2, 50% each to be delivered on 2 consecutive days. In yet another embodiment, a split unit dose may be given on 2 alternate days. In still another embodiment, the unit dose may be split into 3 to be administered equally on 3 consecutive days.

Methods disclosed herein involve administering one or more consecutive doses of cells into a breast duct of a subject who may have received a first dose, and/or administering the first and one or more subsequent doses. The doses are administered in particular amounts and according to particular timing schedule and parameters.

In another aspect, the first dose is administered transpapillarily and any subsequent dose is administered by any suitable means, including transpapillarily, by injection and by infusion, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some embodiments, they are administered by parenteral, intrapulmonary, and intranasal, and, if desired for local treatment, intralesional administration. Parenteral infusions include intramuscular, intravenous, intraarterial, intraperitoneal, intrathoracic, intracranial, or subcutaneous administration.

In some embodiments, the methods generally involve administering the first dose of cells thereby reducing the disease burden. This may be followed by a subsequent dose of cells administered during a particular time of window with respect to the first dose or the administration of the subsequent dose to a subject having received a first dose. The first dose in some embodiments is relatively low. The number of cells administered and the timing of the doses of cells are designed to improve one or more outcomes, such as reduce the likely or degree of toxicity such as CRS, MAS, TLS, neurotoxicity, and the like.

In some embodiments, disclosed herein are methods involving administration of subsequent doses of cells at an increased number, and thus a higher dose, than the first/initial dose.

Where dosing regimen involves multiple doses, each dose may be administered daily, alternate days, every 2 days, 3 days, 5 days, 7 days, 14 days, 15 days, 28 days, monthly, quarterly, 6 monthly, annually.

In some aspects, the timing of doses following initial dose is measured from the initiation of the initial (first) dose to the initiation of the next dose. In other embodiments, the timing of doses following initial dose is measured from the completion of the initial (first) dose.

The present invention encompasses the initial dose may be a split unit dose followed by a second dose administered thereafter. In some embodiments, a second or a subsequent dose may be a split unit dose. By way of a non-limiting example, a split unit dose may be administered over three days and the second unit dose is administered the very next day or it may be administered a year later. Initial dose is intended to create any limitations with regards to a subject in need of such a dose by imply that the subject has never before received a dose of cell therapy or even that the subject has not before received a dose of the same cells expressing the same recombinant receptor or targeting the same antigen.

Generally, cells or compositions comprising cells such as modified cells as described herein may be administered at a unit dose ranging from 1×10³ to 5×10⁸ modified cells/kg body weight, from 0.5×10⁵ to 1×10⁷ modified cells/kg body weight, from 1×10⁴ to 0.5×10⁶ modified cells/kg body weight, from 0.5×10⁴ to 1×10⁶ modified cells/kg body weight, from 1×10⁵ to 0.5×10⁶ modified cells/kg body weight each inclusive. The first dose may be less than 1×10³ cells/kg, less than 0.5×10⁴ cells/kg, less than 1×10⁴ cells/kg, less than 0.5×10⁵ cells/kg, less than 1×10⁵ cells/kg, less than 0.5×10⁶ cells/kg, less than 1×10⁶ cells/kg, less than 0.5×10⁷ cells/kg, less than 1×10⁷ cells/kg, less than 0.5×10⁸ cells/kg, less than 1×10⁸ cells/kg, less than 5×10⁸ cells/kg.

In some aspects, the first dose is a low dose, such as less than 1×10³ cells/kg, less than 0.5×10⁴ cells/kg, less than 1×10⁴ cells/kg, less than 0.5×10⁵ cells/kg, less than 1×10⁵ cells/kg, less than 0.5×10⁶ cells/kg, or less than 1×10⁶ cells/kg. In at least some embodiments, the first dose is less than 1×10⁵ cells/kg.

In other embodiments, the first dose is a high dose, such as greater than 0.5×10⁵ cells/kg, greater than 1×10⁵ cells/kg, greater than 0.5×10⁶ cells/kg, greater than 1×10⁶ cells/kg, greater than 0.5×10⁷ cells/kg, greater than 1×10⁷ cells/kg, greater than 0.5×10⁸ cells/kg, greater than 1×10⁸ cells/kg, greater than 5×10⁸ cells/kg.

In some embodiments, e.g., where risk of toxicity and/or disease burden is determined to be low, the first dose can be a relatively high dose of cells, such as such as greater than 0.5×10⁵ cells/kg, greater than 1×10⁵ cells/kg, greater than 0.5×10⁶ cells/kg, greater than 1×10⁶ cells/kg, greater than 0.5×10⁷ cells/kg, greater than 1×10⁷ cells/kg, greater than 0.5×10⁸ cells/kg, greater than 1×10⁸ cells/kg, and greater than 5×10⁸ cells/kg. Such cells may be modified cells expressing one or more recombinant receptor such as a CAR or engineered TCR, Such modified cells may be T-cells, NK cells, CTLs, monocytes, granulocytes, or progenitors thereof. In at least one embodiment, the first dose can be a relatively high dose, such as greater than 1×10⁷ cells/kg body weight.

In some embodiments, e.g., where risk of toxicity and/or disease burden is determined to be high, the first dose can be a relatively low dose of cells, such as less than 1×10³ cells/kg, less than 0.5×10⁴ cells/kg, less than 1×10⁴ cells/kg, less than 0.5×10⁵ cells/kg, less than 1×10⁵ cells/kg, less than 0.5×10⁶ cells/kg, and less than 1×10⁶ cells/kg. Such cells may be modified cells expressing one or more recombinant receptor such as a CAR or engineered TCR. Such modified cells may be T-cells, NK cells, CTLs, monocytes, granulocytes, or progenitors thereof. In at least one embodiment, the first dose can be a relatively low dose, such as less than 1×10⁵ cells/kg body weight.

In some embodiments, the first dose is large enough to be effective in reducing disease burden. In some embodiments the first dose is large enough to expand *in vivo* and debulk disease. In at least one embodiment, the first dose is large enough to reduce tumor burden. In another embodiment, the first dose reduces tumor size.

In some embodiments, the numbers and/or concentrations refer to numbers of modified cells expressing a chimeric receptor. In other embodiments, the numbers and/or concentrations refer to numbers of all cells administered.

In some embodiments, where the first dose is relatively high, subsequent doses can be lower than the first dose. In other embodiments, where the first dose is relatively low, subsequent doses can be higher than the first dose. In yet other embodiments, doses subsequent to the initial dose can be progressively higher doses. In still other embodiments, the second dose is higher than the initial dose and the subsequent doses remain the same as the second dose. In further embodiments, where the first dose is relatively higher dose, the subsequent doses can be progressively lower doses.

### Side Effects and Toxic Outcomes

Administration of adoptive cell therapy such as treatment with modified cells expressing chimeric receptors can induce severe toxic outcomes or side effects such as cytokine release syndrome (CRS), macrophage activation syndrome (MAS), tumor lysis syndrome (TLS), neurotoxicity, and/or host immune response against the cells and/or recombinant receptors being administered (Bonifant et al., Mol. Ther. - Oncolytics (2016) 3, 16011). Symptoms of CRS, such as fever and increase CRP protein levels appear soon after a first dose within few hours together with increased expression of cytokines such as tumor necrosis factor alpha (TNFα), IFNy, IL-1β, IL-2, IL-6, IL-8, and IL-10. Treatment generally includes anti-IL-6 treatment.

In some aspects, the size of the initial dose and/or subsequent doses is determined based on one or more criteria such as response of the subject to prior treatment for e.g. chemotherapy, disease burden, tumor load, bulk size or degree, extent and type metastasis, stage and likelihood of toxic side effects, such as CRS-related, MAS-related, TLS-related, neurotoxicity-related, and/or host immune response-related outcomes against the cells and/or recombinant receptors being administered. When a subject has a large disease burden, the subject may be administered a low dose of a composition comprising modified cells. When a subject has a low disease burden, the subject may be administered a larger dose of a composition comprising modified cells. Further, the dose may vary depending on tumor burden.

In an aspect, the transpapillary methods disclosed herein increase the cell exposure over time reducing toxic outcomes. Initial administration of high doses need not necessarily result in higher efficacy in subjects with high disease burden, for example, high tumor burden. High doses also need not translate into persistence of the administered cells. Transpapillary methods disclosed herein offer advantages over other methods aimed at reducing the risk of toxic outcomes and/or improving efficacy.

In some embodiments, the methods include administering an initial dose of modified cells expressing a recombinant receptor, for example, a CAR that can expand in the presence of a target antigen and reduce disease burden and/or lower toxic outcomes. Such expansion can be local in the breast duct or lobule or close to the affected tissue. Subject condition can be monitored following administration of an initial dose.

Generally, a subsequent dose may be administered between 7 and 28 days (each inclusive) after the initiation of the first dose, each inclusive. For example, at least 7, 8, 9, 10, 11, 12, 13, 14, 15, 16, 17, 18, 19, 20, 21, 22, 23, 24, 25, 26, or 27 days following the initiation of the first or prior dose or greater than 14 or 15 days or 21 days following the initiation of the first or prior dose. In some embodiments, the timing of a subsequent dose will be determined by risk of side effect or a toxic outcome such as CRS, MAS, TILS, neurotoxicity, host immune response (humoral or adaptive) and the like. The timing of a subsequent dose will be determined by monitoring and/or assessing the presence of one or more of the symptoms, side effects, toxicity outcomes, and/or host immune response, and the acceptable level of such a symptom, side effect, toxic outcome, host immune response and the like. Subject will be administered a subsequent dose after such a symptom, side effect, toxic outcome, host immune response and the like is at an acceptable level. In some embodiments, a subsequent dose may be administered before the subject mounts a host immune response to the first dose or before a host immune response is detectable or reached a certain level, degree or stage. In some embodiments, host immune response (humoral or adaptive) is not detectable before 28 days, 35 days, or 42 days following the first dose of modified cells. In other embodiments, a subsequent dose is administered within 28 days or within 35 days following the first dose or prior dose or before 24, 25, 26, or 27 days following the initiation of the first or prior dose.

In some embodiments, first dose comprises cells in amounts sufficient to reduce disease burden and a subsequent dose is administered at a time when the serum level of a factor indicative of CRS in the subject is no more than 10 or no more than 25 times the serum level of the indicator immediately prior to the first dose, and/or at a time after a CRS-related outcome has reached its peak levels and begun to decline following administration of the first dose and at which the subject does not exhibit detectable adaptive host immune response specific to the recombinant receptor expressed by the modified cells of the first dose.

### Cell exposure and persistence

In some embodiments, the amount and/or timing of a dose is/are designed to promote exposure of the subject to the cells, such as by promoting their expansion and/or persistence over time. In some embodiments, the transpapillary methods provided herein reduce migration period for infiltrating the affected breast tissue. In some embodiments, the transpapillary methods provided herein increase the exposure of the subject to the administered cells or compositions and/or improve their efficacy and therapeutic outcomes in adoptive cells therapy. In some aspects, the greater and faster exposure to the modified cells improves the treatment outcomes as compared with other methods. Such outcomes may include patient survival and remission and/or reduced toxic outcomes.

In an aspect, modified cells may be tagged with a contrast agent such as gadolinium-based agents such as gadolinium chelates, gadolinium fullerenol, superparamagnetic iron oxide nanoparticles (SPION), ¹⁹F perfluorocarbon nanoparticles, and other magnetic reporter genes, such as metalloprotein-based MRI probes. This would allow the tracking of the modified cells migration and localization of the modified cells. In some embodiments, modified cells are tagged with a contrast agent such gadolinium chelates, superparamagnetic iron oxide nanoparticles (SPION), ¹⁹F perfluorocarbon nanoparticles, or other magnetic reporter genes, such as metalloprotein-based MRI probes. In at least one embodiment, the contrast agent is a gadolinium chelate. Gadolinium chelates are clinically approved contrast agents that be been used to labels cells in experimental cellular MRI studies. They may be loaded into cells by methods known in the literature, for example, by electroporation.

In some embodiments, the presence and/or amount of cells expressing the recombinant receptor, for example, a CAR, in the subject following the first dose and/or following the subsequent dose(s) is detected. Presence and/or amount of cells expressing the recombinant receptor may be detected by any of the methods known in the art. For example, detection may be by quantitative PCR (qPCR) assay or next generation sequencing methods to measure the copies of DNA or plasmid encoding the receptor/microgram of DNA or as the number of modified cells/microliter of the sample (for example, blood, serum, nipple aspirate fluid, per total number of PBMCs) or by cell-based assays such as ELISA directed to the modified cells expressing the recombinant receptor. In some embodiments, at least 50, at least 100, at least 500, at least 1×10³, at least 0.5×10⁴, at least 1×10⁴, at least 0.5×10⁵, at least 1×10⁵, at least 0.5×10⁶, at least 1×10⁶ or at least 1×10⁷, at least 1×10⁸ cells are detectable.

In some embodiments, the persistence of cell expressing the receptor, e.g., CAR, in the subject by methods following the subsequent dose(s), and/or following administration of the first dose, is greater as compared to those compared administered by other routes of delivery known in the art. In some embodiments, the persistence of cell expressing the receptor, e.g., CAR, in the subject by methods following the subsequent dose(s), and/or following administration of the first dose, is greater as compared to those administered by an alternate dosing regimen. In some embodiments, the migration and/or infiltration of the administered cells into the breast tissue, for example, the affected tissue, may be detected by MRI scan or by excision of the breast tissue by mastectomy or lumpectomy post-surgery by immunohistochemistry.

In another aspect, increased exposure of the subject to administered cells includes increased *in vivo* expansion of the cells as measured by flow cytometry quantification of cells present in nipple fluid collected by aspiration or ductal lavage. Devices (for example, Forecyte^{®} and Fullcyte) and methods for collection of nipple aspirate or ductal lavage fluids have been published and described (US6,689,070; US6,585,706).

In some embodiments, the modified cells are detectable in the breast duct at least 15, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 days or more following administration of the first dose, or after administration of a subsequent dose for at least 4, 5, 6, 7, 8, 9, 10, 11, 12, 13, 14, 15, 20, 21, 22, 23, 24 weeks.

### Cells

Cells that may be used for adoptive cell therapy for the purpose of the present invention, include genetically engineered cells (modified cells) and unmodified immune cells, for example peripheral blood mononuclear cells (PBMCs), T-cells, NK cells, CTLs, TILs, progenitors thereof, and like.

A "modified cell" as used herein is a genetically engineered cell that expresses at least one recombinant receptor designed to recognize and/or specifically bind to a target molecule associated with a disease or a condition such as a breast disorder, and result in a response, such as an immune response against such molecules upon binding to such molecules.

Modified cells for the purpose of this invention are generally eukaryotic cells, such as mammalian cells. Typically, the mammalian cells are human cells. In some embodiments, the cells are derived from blood, bone marrow, lymph or lymphoid organs. The cells are typically cells from the immune system, such as those of adaptive or innate immunity. Such cells may be lymphoid cells or myeloid cells, including, T-lymphocytes (T-cells), natural killer cells (NK cells), tumor infiltrating lymphocytes (TILs), cytotoxic lymphocytes (CTLs), and progenitors thereof. The cells may be stem cells such as multipotent and pluripotent stem cells, including inducible pluripotent stem cells (iPSCs). The cells are typically primary cells, such as those isolated from a subject, and/or those isolated from a subject and frozen.

Modified cells may include one or more of the subsets of T-cells or other cell types, such as whole T-cell populations, CD4+, CD8+ T-cells and subpopulations thereof. Subpopulations of T-cells and/or of CD4+ and/or of CD8+ T-cells include naive T-cells cells, effector T-cells, memory T-cells and subtypes thereof (such as stem cell memory T-cells, central memory T-cells, effector memory T-cells, or terminally differentiated memory T-cells), tumor-infiltrating T-cells (TILs), immature T-cells, mature T-cells, helper T-cells (such as T_{H1}, T_{H2} and T_{H3}, T_{H17}, T_{H19}, T_{H22} cells, follicular helper T-cells), cytotoxic T-cells (CTLs), mucosa-associated invariant T-cells (MATT cells), naturally occurring and adaptive regulatory T-cells (T-regs), alpha/beta T-cells and delta/gamma T-cells. Modified cells may also include TCR-deficient T-cells.

The subpopulation of the cells may be defined by function, activation, state, maturity, potential for differentiation, expansion, recirculation, localization, and/or persistence capacities, antigen-specificity, presence in a particular organ or compartment, marker or cytokine secretion profile, and/or degree of differentiation. In some embodiments, modified cells comprise those that tend to localize in the breast tissue.

In some embodiments, the modified cells are T-cells. In other embodiments, the modified cells are CTLs. In still other embodiments, the modified cells are natural killer (NK) cells. In yet other embodiments, cells are monocytes or granulocytes, e.g., myeloid cells, macrophages, neutrophils, dendritic cells, mast cells, eosinophils, and/or basophils. Modified cells can also be progenitors cells of any of the cells disclosed herein. In some embodiments, the modified cells are progenitor cells of T-cells, NK cells, CTLs, monocytes, and granulocytes.

With reference to a subject, modified cells administered transpapillarily can be autologous, heterologous (allogeneic), or xenogeneic. "Autologous" as used herein refers to any material (such as cells) derived from the same individual to whom it is later to be re-introduced into the individual. As used herein "heterologous" and "allogeneic" refers to any material (such as cells) derived from a different animal of the same species as the individual to whom the material is introduced. Two or more individuals are said to be allogeneic to one another when the genes at one or more loci are not identical. In some aspects, allogeneic material from individuals of the same species may be sufficiently unlike genetically to interact antigenically. Some cells may be "off-the shelf" or "universal" donor cells obtained from healthy donors (Torikai et al., Blood 2012 119:5697-5705). As used herein, "xenogeneic" refers to a material (such as cells) derived from an animal of a different species.

In some embodiments, the cells may be derived from cells lines, e.g. T-cell lines and NK cell lines. The cells in some embodiments are from xenogeneic source, for example, from pig, mice, rats, and non-human primates. In other embodiments, the cells may be derived from cord blood.

The cells are isolated from a circulating blood and are harvested, e.g., by non-affinity based techniques such as apheresis or leukapheresis and density-based cell separation methods (e.g., percoll or ficoll gradient centrifugation), by affinity or immunoaffinity based techniques such as separation by expression or presence on the cells of one or more specific molecules e.g., cell surface molecules. In case of affinity-based separation, the separation can be positive selection (cells are bound to its partner are retained) or negative selection in which the cells have not bound to the antibody or its binding partner and are retained. The latter is useful if antibodies to the marker are unavailable. Separation need not result in 100% enrichment or removal of particular cell population(s). One or more rounds of separation may be carried out to obtain the desired enriched population. Binding partners or antibody conjugated to magnetic beads for example DYNABEADS or MACS beads, may be utilized for separation and enrichment of cells. Separation and/or enrichment may also be performed by flow cytometry, FACs, fluidics, MEMs methods. Cell selection may be further performed using ExPact Treg beads, TransAct Beads, Expamer and/or using cell-based T-cell activation such as antigen-presenting cells (APCs) such as dendritic cells, artificial APCs, etc. Methods for clinical manufacturing of cells have been described by Wang and Riviere in Molecular Therapy - Oncolytics (2016) 3, 16015, which is incorporated by reference in its entirety in the present application.

Multiple cell types may be simultaneously selected by use of multiple binding partners or antibodies in a single step. For example, in some aspects, specific subpopulations of T-cells such as cells expressing high levels of one or more cell surface markers such as CD3+, CD4+, CD8+, CD27+, CD28+, CD45RA+, CD45RO+ and/or CCR7+ may be isolated by positive or negative affinity based selection methods.

In some embodiments, CD4+ helper cells are enriched and sorted into naive (with CD45RO /CD45RA+ /CD62L+ antigens), central memory (with CD62L+/CD45RO+ antigens) and effector cells (with CD62L-/CD45RO- antigens) by identifying cell populations that have the appropriate cell surface antigens.

### Recombinant Receptor

Modified cells express recombinant receptors that include chimeric receptors, e.g., chimeric antigen receptors (CARs), and other transgenic antigen receptors, including disease-specific T cell receptors (TCRs) and engineered TCRs such as transgenic TCRs. In one aspect, modified cells disclosed herein can include one or more recombinant receptors. For example, a modified cell may express a first CAR, a second CAR, a third CAR and so forth, each of which can be independently monospecific, bispecific or multispecific. In at least one embodiment, a modified cell may express at least two chimeric receptors, each chimeric receptor recognizing a different target antigen.

In another aspect, modified cells that are transpapillarily expressed may include cells that are a mixture of cells that express different recombinant receptors, for example, modified cells include a first cell expressing a HER2-CAR and a second cell expressing a FAP-CAR or PD-1-CAR. The expression of the recombinant receptors may be constitutive, inducible, transient or switchable. In one aspect, recombinant receptors, including CARs and engineered or disease-specific TCRs, comprises at least one chimeric recombinant protein comprising at an extracellular antigen binding domain, a transmembrane domain and an intracellular signaling domain comprising a functional signaling domain.

TCRs that are disease-specific, for example, breast cancer specific, may be isolated from a rare tumor-active T-cell or, where this is not possible alternative technologies may be employed to generate highly active antitumor T-cell antigens. In some embodiments, transgenic mice may be immunized to generate T-cells expressing TCRs directed against HLA-restricted human tumor antigens (Stanislawski et al. Nat. Immunol. 2001, 2, 962 - 970).

In some embodiments, allogenic TCR gene transfer in which tumor-specific T-cells are isolated from a patient experiencing tumor remission and the reactive TCR sequences are transferred to T-cells from host T-cells who shares the disease but is non-responsive (Gao et al., Blood. 2000, 95, 2198-2203; de Witt et al., 2006, Blood, 108, 870-877). In other embodiments, in vitro technologies can be employed to alter the sequence of TCR, enhancing their tumor-killing activity by increasing the strength of the interaction (avidity) of a weakly reactive tumor-specific TCR with target antigen (Robbins et al., 2008, J. Immunol. 180, 6116-6131). In some embodiments, modified cells expressing TCRs lack self-TCR, i.e., TCRs were transferred to cells that are TCR deficient.

As used herein, the terms "Chimeric Antigen Receptor" and a "CAR" refer to a recombinant polypeptide construct comprising an extracellular antigen binding domain (Ag-binding domain), a transmembrane (Tm) domain and a intracellular cytoplasmic signaling domain comprising a functional signaling domain derived from a stimulatory molecule as defined below. CARs combine both antibody-like recognition with T-cell activating function.

A recombinant receptor such as a CAR or engineered or disease-specific TCR may target a cell surface molecule, an intracellular antigens (for example, via HLA-restricted presentation), or both.

After Ag-binding domain of a recombinant receptor (for example, of a CAR) binds to its target antigen, the cytoplasmic signaling domain activates intracellular signaling that induce persistence, trafficking, and effector functions. For example, the Ag-binding domain can redirect T-cell specificity and reactivity toward a selected target in a non-MHC restricted manner, exploiting the antigen-binding properties of antibodies. The non-MHC-restricted antigen recognition gives T-cells expressing a CAR the ability to recognize an antigen independent of antigen processing, thus bypassing a major mechanism of tumor escape. Moreover, when expressed in T-cells, CARs provide an added benefit of not dimerizing with endogenous TCR alpha and beta chains.

### Ag-Binding Domain

Sequences used to define the antigen targeting motif for a recombinant receptor (as an illustrative example, a CAR) are typically derived from a monoclonal antibody, but ligands may also be used. For example, a HER2-CAR. Ag-binding domain can be derived from any of the monoclonal antibodies that bind to HER2, such as 4D5 (Herceptin) In one aspect, extracellular antigen binding domains (Ag-binding domains) recognize and bind target antigens. The Ag-binding domain may include Ag-binding domain of an antibody. Such an Ag-binding domain of an antibody may comprise a portion of an antibody molecule, generally a variable heavy (V_{H}) chain region and/or variable light (V_{L}) chain region of the antibody or antibody fragment, e.g., an scFv antibody fragment.

The term "antibody," as used herein, refers to an immunoglobulin molecule which specifically binds with an antigen. Antibodies can be polyclonal or monoclonal, multiple or single chain, or intact immunoglobulins, and may be derived from natural sources or from recombinant sources. Antibodies such as IgG are typically tetramers of immunoglobulin molecules.

The term "antibody fragment" refers to at least one portion of an intact antibody, or recombinant variants thereof, and refers to the antigen binding domain, e.g., an antigenic determining variable region of an intact antibody, that is sufficient to confer recognition and specific binding of the antibody fragment to a target, such as an antigen. Examples of antibody fragments include, but are not limited to, Fab, Fab', F(ab')2, and Fv fragments, scFv, linear antibodies, single domain antibodies such as sdAb (either VL or VH), camelid VHH domains, and multispecific antibodies formed from antibody fragments. The term "scFv" refers to a fusion protein comprising at least one antibody fragment comprising a variable region of a light chain and at least one antibody fragment comprising a variable region of a heavy chain, wherein the light and heavy chain variable regions are contiguously linked via a short flexible polypeptide linker, and is capable of being expressed as a single chain polypeptide, and wherein the scFv retains the specificity of the intact antibody from which it is derived. Unless specified, as used herein an scFv may have the VL and VH variable regions in either order, e.g., with respect to the N-terminal and C-terminal ends of the polypeptide, the scFv may comprise VL-linker-VH or may comprise VH-linker-VL.

In some embodiments, the Ag-binding domain is an scFV antibody fragment.

The Ag-binding domain of a recombinant receptor such as a CAR may be monospecifc, bi-specific or multispecific (i.e., bind 3 or more different antigens) or any combination thereof. The ScFv, the Fab, Fab2, Fab' may comprise epitopes derived from an antibody targeting a desired target antigen such as HER2 and accordingly, the scFV, Fab, Fab2, Fab' may comprise epitopes anti-HER2/neu antibody trastzumab that recognize and bind HER2 on a breast cell.

In one aspect, Ag-binding domain may be monospecific, bispecific, or multispecific, meaning that an Ag-binding domain might bind a single target antigen, 2 target antigens or 3 or more target antigens. Accordingly, the present invention encompasses Ag-binding domains of a recombinant receptor such as a CAR that comprise one or more target antigen binding sequences in tandem. As a non-limiting example, a bi-specific Ag-binding domain of a recombinant receptor such as a CAR can be any ScFv further comprising sequences from anti-HER2 and andti-IL-13Ralpha in tandem and is capable of binding both HER2 and IL-13. Trispecific monoclonal antibodies and methods of making them have been described (Castoldi et al., Protein Engineering, Design, and Selection (2012) vol. 25(10): 551-559; Wang et al., J Biochem. 2004 Apr;135(4):555-65; Dimasi et al., Mol. Pharmaceutics, 2015, 12 (9), pp. 3490-3501). As another illustrative example, a trispecific Ag-binding domain can be an ScFv or a Fab domain comprising sequences derived from a trispecific monoclonal antibody targeting ErbB2 (HER2), c-Met, and IGF1R. Such bi-specific and multispecific Ag-binding domain containing recombinant receptors are highly desirable for greater tumor specificity.

In another aspect, the Ag-binding domain of a recombinant receptor, such as a CAR, comprises one or more HLA-restricted epitopes.

### Leader

Ag-binding domains of a recombinant receptor can further comprise a leader sequence at the N-terminus of the Ag-binding domain. The leader sequence may be cleaved from the Ag-binding domain, for example, from the scFv domain, during cellular processing and localization of the CAR to the cellular membrane. In some embodiments, the leader sequence is cleaved from the Ag-binding domain (and thereby the chimeric receptor, for example, from the CAR). In at least one embodiment, the leader sequence is not cleaved and remains a part of the Ag-binding domain. The retained leader sequence does not disturb functionality of the Ag-binding domain, and thus, of the recombinant receptor.

### Spacer/hinge

In some embodiments, the Ag-binding domain a recombinant receptor such as a CAR may further include a spacer. The spacer may include at least a portion of an immunoglobulin constant region (constant domain) or a variant or a modified version thereof, such as a hinge region. Examples of such hinge regions are known in the art (e.g., IgG1 or IgG4 hinge, CH1/CL, etc.). In at least one embodiment, the hinge region is a human hinge region, such as human IgGl, IgG2, IgG3 or IgG4. In another embodiment, the spacer is a humanized version of hinge regions. In another embodiment, the spacer is the constant domain between the antigen recognition sites and the transmembrane domain.

Spacer may be of any length suitable that provides capability for a cell to respond upon antigen binding. A spacer may comprise up to 300 amino acids. In some embodiments, the spacer is 10 to 150 amino acids. In other embodiments, the spacer is 15 to 50 amino acids. In yet other embodiments, the spacer is 1 to 10 amino acids.

Exemplary spacers include, but are not limited to IgG4 hinge alone, IgG1 hinge alone, IgG4 linked to CH2 and CH3 domains or IgG4 linked to CH2 domain alone or IgG4 linked to CH3 domain alone. In some embodiments, the constant domain or portion thereof of a human IgG (such as IgG1 or IgG4), IgM or IgA. Additional spacers are known in the art (*See*, Hudeek et al. (2013). Clin. Cancer Res. 19:3153; WO2014031687; US8822647, US20140271635).

### Transmembrane Domain

In some embodiments, the transmembrane (Tm) domain of a recombinant receptor such as a CAR is fused with the Ag-binding domain. In other embodiments, the Tm domain is separated from the Ag-binding domain by a spacer.

The Tm domain may be derived from a natural source or from a synthetic source. When the Tm domain is derived from a natural source, it may be from any membrane-bound or transmembrane protein. By way of non-limiting examples, proteins such as alpha, beta or zeta chains of a T-cell receptor (TCR), CD28, CD3, CD3e, CD45, CD4, CD5, CDS, CD9, CD16, CD22, CD33, CD37, CD64, CD80, CD86, CD134, CD154 and transmembrane regions containing functional variants thereof are useful for the purpose of the present invention. In some embodiments, the Tm domain is a Tm domain of CD4, CD28, CD8 or functional variants thereof. It will be understood by a person skilled in the art that mutations and other modifications of Tm domain sequences derived from nature sources are contemplated to be within the present invention.

In at least one embodiment, the Tm domain is synthetic. Synthetic transmembrane domains are known and described in the art (US7052906). In some aspects, synthetic Tm domain comprises predominantly hydrophobic residues such as valine and leucine, leucine-zippers.

In other embodiments, the Tm domain is linked to a signaling domain by a linker or a spacer. The linker can be of any length suitable for intracellular signaling. In some embodiments, the linker is of length 1 - 15 amino acids in length.

### Signaling domain

The recombinant receptor, e.g., a CAR, typically includes at least one intracellular signaling domain. Intracellular signaling domains can include those that mimic a signal through a natural antigen receptor alone, a signal through an antigen receptor in combination with one or more co-stimulatory receptor, or a signal through a co-stimulatory receptor alone. In some embodiments, the signaling domain comprises a primary stimulatory molecule. A "primary stimulatory molecule" and "primary stimulatory domain" used interchangeably herein in the context of recombinant receptor such as a CAR, means a molecule or a portion, variant or a modification thereof expressed by a modified cell that provides the primary cytoplasmic signaling sequence(s) that regulate primary activation of the TCR complex in a stimulatory way for at least some aspect of the T-cell signaling pathway.

In one aspect, the primary signal is initiated by, for instance, binding of a TCR/CD3 complex with an MHC molecule loaded with peptide, and which leads to mediation of a T-cell response, including, but not limited to, proliferation, activation, differentiation, cytokine release, and the like. The primary cytoplasmic signaling domain that acts in a stimulatory manner or induces stimulation may include, without limitation, those derived from immunoreceptor tyrosine activation motifs (ITAMs) such as those derived from TCR zeta, FcR gamma, FcR beta, CD3 gamma, CD3 delta, CD3 epsilon, CD4, CD8, CD16, CD22, CD25, CD79a, CD79b, and CD66d. Stimulatory molecules provided herein are listed merely by way of example. The list is not intended to be exclusive and further examples will be readily apparent to those of skill in the art.

In some embodiments, the recombinant receptor, e.g., a CAR, includes an intracellular component of TCR complex, such as CD3 chain that mediates T-cell activation and cytotoxicity, for example CD3 zeta chain. Thus, in at least one embodiment, the stimulatory molecule is the CD3zeta chain associated with the T cell receptor complex. In some embodiments, the receptor comprises CD3 Tm domain and CD3zeta stimulatory domain. In other embodiments, the receptor further comprises Fc receptor gamma.

While not intending to be bound by any particular theory of operation, by the term "stimulation," used in the context of a modified cell, is meant a response induced by binding of a stimulatory molecule (e.g., a TCR/CD3 complex) with its cognate ligand thereby mediating a signal transduction event, such as, but not limited to, signal transduction via the TCR/CD3 complex. Stimulation can mediate altered expression of certain molecules, such as downregulation of TGF-beta., and/or reorganization of cytoskeletal structures, and the like

Where the modified cell is a T-cell, full activation requires not only signally through its TCR complex, but also requires a co-stimulatory signal that is antigen nonspecific, usually provided by co-stimulatory molecules expressed on the membranes of antigen presenting cells and T-cells.

In an aspect, the recombinant receptors include one or more co-stimulatory domains. First generation of CAR-T cells included a single co-stimulatory domain. The second generation CAR-T cells include two stimulatory domain, and the third generation CAR-T cells include multiple stimulatory domains. Accordingly, in some embodiments, the modified cells include one or more co-stimulatory domains. In some embodiments, the cytoplasmic signaling domain further comprises functional signaling domains derived from at least one costimulatory molecule. A "costimulatory molecule" refers to the cognate binding partner on a modified cell that specifically binds with a "costimulatory ligand" as defined below, thereby mediating a costimulatory response by the modified cell, such as, but not limited to, proliferation. Costimulatory molecules typically are cell surface molecules other than target antigen receptors or their ligands that are required for an efficient immune response.

In some embodiments, the stimulatory domain is included in one recombinant receptor, such as a CAR, and the costimulatory domain is provided by a second recombinant receptor such as CAR recognizing a second target antigen. In other embodiments, chimeric receptors, such as CARs comprising primary stimulatory domain and chimeric receptors, such as CARs comprising costimulatory domain are expressed in the same modified cell. In yet other embodiments, the stimulatory domain and one or more co-stimulatory domain are in tandem when expressed on the same modified cell.

Costimulatory molecules for the purposes of the present invention include, but are not limited to an MHC class I molecule, BTLA and a Toll ligand receptor, immunoglobulin superfamily (IgSF) such as CD28, B7 receptor family members (B7-H2ZB7RP-1ZLICOS/GL50, B7-DC/PD-L2, B7-H3), CD226, TIM, CD2/SLAM, BTN, LAIR, tumor necrosis factor receptor superfamily (TNFRSF) such as OX40, CD27, CD30, DR3, GITR, and HVEM, CD2 and SLAM on T-cells, ICAM-1, LFA-1 (CD11a/CD18), adhesion molecules (CD54, CD58, CD70), ICOS, CD40, CD40L, 4-1BB (CD137), CD70, CD80, CD86, DAP10, and other orphan receptor families such as LAG3 (CD223) and CD160.

Accordingly, in some embodiments a costimulatory molecule is selected from the group consisting of MHC class I molecule, BTLA and a Toll ligand receptor, immunoglobulin superfamily (IgSF) such as CD28, B7 receptor family members (137-H2/B7RP-1/LICOS/GL50, B7-DC/PD-L2, B7-H3), CD226, TIM, CD2/SLAM, BTN, LAIR, tumor necrosis factor receptor superfamily (TNFRSF) such as OX40, CD27, CD30, DR3, GITR, and HVEM, CD2 and SLAM on T-cells, ICAM-1, LFA-1 (CD11a/CD18), adhesion molecules (CD54, CD58, CD70), ICOS, CD40, CD40L, 4-1BB (CD137), CD70, CD80, CD86, DAP10, and other orphan receptor families such as LAG3 (CD223) and CD160. Co-stimulatory molecules provided herein are listed merely by way of example. The list is not intended to be exclusive and further examples will be readily apparent to those of skill in the art.

A "costimulatory ligand" refers to a molecule that provides a non-antigen-specific signal important for full activation of an immune cell (e.g., T-cell). Costimulatory ligands include, without limitation, tumor necrosis factor (TNF) ligands, immunoglobulin superfamily (IgSF) ligands and cytokines (such as IL-2, IL-12, IL-15, and IL21). TNF is a cytokine involved in systemic inflammation and stimulates acute phase reaction. Its primary role is in the regulation of immune cells, TNF ligands share a number of common features: the majority of the ligands are synthesized as type II transmembrane proteins (extracellular C-terminus) contains a short cytoplasmic segment and relatively long extracellular region. The TNF ligands include but are not limited to nerve growth factor (NGF), CD40L, CD40L/CD154, CD137L/4-1BBL, TNF-α, CD134L/OX40L/CD252, CD27L/CD70, Fas ligand (FasL), CD30L/CD153, TNF-beta (TNFβ)/lymphotoxin-alpha (LTα), lymphotoxin-beta (LTβ), CD257/B cell-activating factor (NAFF)/Blys/THANK/Tall-1, glucocorticoid-induced TNF Receptor ligand (GITRL), and TNF-related apoptosis-inducing ligand (TRAIL), LIGHT (TNFSF14) and SLAM. The Ig superfamily is a large group of cell surface and soluble proteins that are involved in the recognition, binding, or adhesion process of cells. These proteins share structural features with immunoglobulins such as an immunoglobulin domain (fold). IgSF ligands include, without limitation, CD80 (B7-1) and CD86 (B7-2), each a ligand for CD28. Additional co-stimulatory molecules/ligands are known in the art (Chen and Files. Nat. Rev. Immunol. 2013 Apr. 13(4): 227-242), incorporated by reference herein in its entirety.

### Target Antigens

In some embodiments, target antigens that bind to the Ag-binding domain of a recombinant receptor, such as CAR or an engineered or disease-specific TCR (such as a tumor-specific TCR) can be cell surface proteins/markers. In other embodiments, the target antigens can be intracellular molecules. Target antigens may be expressed on a breast cell, preferably on a breast cell affected by a disease such as breast cancer. Some antigen target antigens may also be expressed on non-breast cells and tissues. In one embodiment, the Ag-binding domain of a chimeric receptor can be engineered to target an antigen associated with breast disease such as breast cancer. Biomarkers referred to anywhere in the present disclosure and published in literature can be target antigens for the purpose of this invention. The antigens provided herein are listed merely by way of example. The list is not intended to be exclusive and further examples will be readily apparent to those of skill in the art. The selection of antigen will depend on the particular type of disease, for example, the type of breast cancer, to be treated. Tumor antigens are proteins produced by tumor cells that elicit an immune response, particularly T-cell mediated immune response.

The target antigen comprises one or more antigenic epitopes, such as antigenic cancer epitopes, associated with a malignant tumor. Malignant tumors express a number of proteins that can serve as target antigens for an immune attack. Another group of target antigens are oncofetal antigens such as cancinoembryonic antigen (CEA). The type of tumor antigens referred to in the invention also include tumor-specific antigen (TSA) and tumor-associated antigen (TAA). A TSA is unique to tumor cells and is not expressed on other cells in the body. A TAA antigen is not unique to tumor cells and may be expressed on normal cells under conditions that prevent the development of immunologic tolerance to the antigen and tumor escape. But the presence of TAA on a tumor cells can permit the development of immunologic tolerance and tumor escape. TAAs may be antigens that are expressed on normal cells during fetal development when the immune system is immature and unable to respond or they may be antigens that are normally present at extremely low levels on normal cells but which are expressed at higher levels on tumor cells.

Non-limiting examples of TSA or TAA include tumor-specific multilineage antigens such as MAGE1, MAGE-3, MAGE-A3/6, MAGE-A family members such as MAGE-A1, MAGE-A2, MAGE-A3, MAGE-A4, MAGE-A6, MAGE-A12, MAGE-A9, MAGE-A11, MAGE-C1, and MAGE-C2, RAGE, BCR-ABE, protein tyrosine kinases such as PRL-2 and PRL3, tumor associated glycoproteins such as TAG-72, CA 19-9, CA 27.29, CηA 72-4, CA 50, receptor tyrosine kinases such as H4-RET and the like.

In at least one embodiment, a target antigen for invasive breast cancer can be MAGE-A1, MAGE-A2, MAGE-A3, MAGE-A4, MAGE-A6, and/or MAGE-A12. In some embodiments, the target antigen is MAGE-A3/6. MAGE-A3/6 is attractive for primary breast cancer with hormone receptor (such as estrogen receptor and progesterone receptor) negative status in a subject. In yet other embodiments, MAGE-A9 and MAGE-A11 are desirable target for ER+ breast cancers and HER2+ breast cancers. In some embodiments, desirable targets for the treatment of TNBCs include those described by Lehrmann et al. (J. Clin. Invest. 2011. 121(7), 2750 - 2767).

In at least one embodiment, target antigen is HER2. A HER2-targeting recombinant receptor provided herein (for example, a HER2-CAR) has at least 80%, at least 81%, at least 82%, at least 83%, at least 84%, at least 85%, at least 86%, at least 87%, at least 88%, at least 89%, at least 90%, at least 91%, at least 92%, at least 93%, at least 93%, at least 94%, at least 95%, at least 96%, at least 97%, at least 98%, at least 99%, and 100% homology to SEQ ID NO:1 disclosed in FIGURE 2 or a variant or functional portion thereof. In some embodiments, a HER2-CAR has at least 80% to 100% identity with SEQ ID NO:1 disclosed in FIGURE 2 or a variant or functional portion thereof. In at least one embodiment, a HER2-CAR has at least 95% identity with SEQ ID NO:1 disclosed in FIGURE 2 or a variant or functional portion thereof

In some embodiments, the HER2-CAR comprises sequences derived from the humanized 4D5 anti-HER antibody directed to HER2 epitopes. Various epitopes on HER2 that can be targeted and bound by an Ag-binding domain of a CAR have been described in literature and that are useful for the purpose of the present disclosure. In at least one embodiment, the HER2-CAR binds directly to an epitope on subdomain IV of the extracellular domain of HER2. In some embodiments, the HER2-CAR binds to amino acid residues 557-561, 570-573 and 593-603 on HER2 (Rockberg et al., Molecular Oncology, 3 (2009) 238-247, incorporated by reference herein in its entirety). In other embodiments, the HER2-CAR binds to the epitopes on the extracellular domain of HER2 ((N1:YNTDTFES (SEQ ID NO: 12), N2:NPEGRYTFGA (SEQ ID NO: 13) and N3:VGSCTLVCPLHNQEVT (SEQ ID NO: 14), C1:LPESFDGD (SEQ ID NO: 15) and C2:LQVF (SEQ ID NO: 16)), Id. Additional epitopes that HER2-CAR can bind include those described by Rongcun et al. (J Immunol 1999; 163:1037-1044, incorporated by reference herein in its entirety). In some embodiments, the HER2-CAR comprises a Fab domain or an scFV derived from 4D5 antibody directed to HER2, a CD8 hinge region, a CD8 spacer, co-stimulatory domains CD28 and 4-1BB and CD3z as the intracellular signaling domain.

In still other embodiments, CA 19-9 is a desirable target for early and relapsed ductal breast carcinoma. In another embodiment, Folate Receptor-α (FR-α), mesothelin, ROR1, carbohydrate sequences from prostate cancer-associated antigens are desirable targets for triple negative breast cancer, invasive breast cancers and metastatic breast cancers.

FR-α-targeting recombinant receptors have been published. For example, methods of preparing a FR-alpha-CAR based on Mov18 antibody directed to FR-α have been described previously by Song et al. (Blood. 2012 Jan 19;119(3):696-706; Oncoimmunology. 2012 Jul 1; 1(4): 547-549; J. Hematology & Oncology (2016) 9:56). In some embodiments, a FAP-CAR comprises an scFV derived from the anti-FR-α antibody MOv18 antigen binding domain, a CD8 hinge region, a CD8 Tm region, and a co-stimulatory domain comprising CD27 and a CD3z intracellular domain. Additional Ag-binding domains of a recombinant receptor may be derived from monoclonal antibodies such as 9F3, 24F12, 26B3, 19D4 directed against FR-α (O'Shannessy et al., Oncotarget. 2011 Dec; 2(12): 1227-1243.), monoclonal antibodies IMGN853 (See, US 2012/0282175) and farletuzumab. Various epitopes that FR-α-CAR may bind have been mapped and described (*Id.*) In some embodiments, FR-α-CAR is constitutively, transiently, inducibly, or switchably expressed in modified cells or is conditionally active.

Fibroblast activation protein (FAP) is a desirable target for invasive breast cancer or any cancer involving the breast tissue stroma. A FAP-targeting recombinant receptor provided herein (for example, a FAP-CAR) has at least 80%, at least 81%, at least 82%, at least 83%, at least 84%, at least 85%, at least 86%, at least 87%, at least 88%, at least 89%, at least 90%, at least 91%, at least 92%, at least 93%, at least 93%, at least 94%, at least 95%, at least 96%, at least 97%, at least 98%, at least 99%, and 100% homology to SEQ ID NO:2 disclosed in FIGURE 2 or a variant or functional portion thereof. Methods for making FAP-CARs have been previously described (Kakarla, et al., Mol Ther. (2013), 21 8, 1611-1620; Wang et al., Cancer Immunol Res. 2014 Feb; 2(2): 154-166), June et al. (US patent publication 2014/0099340).

In another aspect, the target antigens include, without limitation, antigens that have immunosuppressive activity. Cells expressing CARs directed to immunosuppressive target antigen are termed inhibitory CARs (iCARs). Exemplary immunosuppressive target antigens include, check-point inhibitors such as PD-1, CTLA-4, CD47 and their ligands. Breast cancers, for example, triple negative breast cancers, have been reported to correlate with poor prognosis in the setting of PD-1 ligand ((PD-1L). Therefore in at least one embodiment, a target antigen includes PD-L1. CARs targeting PD-1, CTLA-4, CD47 and their ligands such as PD-L1 may be monospecific, bi-specific or multispecific. Modified cells expressing antigen-specific inhibitory receptors (termed inhibitory CAR-T cells or iCAR-Ts) are useful to reducing immunosuppression, hostile tumor microenvironment and divert off-target immunotherapy responses. Methods to make iCARs have been described in the art (See, Cherkassy et al., J. Clin. Invest. 2016;126(8):3130-3144; Federov et al., Science Translational Medicine 11 Dec 2013: Vol. 5, Issue 215, pp. 215ra172).

Additional exemplary target antigens recognized by a extracellular Ag-binding domain of a recombinant receptor include, without limitations, transformation-related molecules such as BER2/neu or ErbB-2 (HER2), MUCs such as MUC1, c-met, cytokeratins such as CK5, CK6, CK14, CK7, CK8, CK14, CK17, CK18, CK19, p53, glycosides, Tn, TF, and sialyl Tn (STn), Folate Receptor-alpha (FR-α), ROR1, tumor associated antigens such as carcinoembryonic antigen (CEA), L1 cell adhesion molecule (L1CAM), fibroblast activation protein (FAP), diganglioside GD2, mesothelin, IL-13 receptor IL13R, IL-13 receptor α, ephrinB2, IGFR1, eLIGHT, WT1, TAG-72, Ep-CAM, LFA-1, EGFR, estrogen receptor (ER), progesterone receptor, and the like. Additional antigen receptors and methods for engineering and introducing such receptors into cells, include WO200014257, WO2013126726, WO2012/129514, WO2014031687, WO2013/166321, WO2013/071154, WO2013/123061, US2002131960, US2013287748, US20130149337, US20160206656, US20160045551, US20160158359, US20160151491, US9365641, US8906682 and the like. Further targets and CARs include those described by Sadelain et al., Cancer Discov. 2013, April 3(4):388-398; Davila et al (2013) PLoS One 8(4):e61338; Turtle et al., Curr. Opinion. Immunol. 2012 October; 24(5): 633-39; Wu et al., Cancer, 2012 March 18(2): 160-75, each incorporated by reference in its entirety. In some aspects, the antigen receptors include a CAR as described in U.S. Pat. No. 7,446,190, and those described in International Patent Application Publication No.: WO/2014055668 A1. Examples of the CARs include those disclosed in any of the aforementioned publications, such as WO2014031687, U.S. Pat. No. 8,339,645, U.S. Pat. No. 7,446,179, US 2013/0149337, U.S. Pat. No. 7,446,190, U.S. Pat. No. 8,389,282, Kochenderfer et al., 2013, Nature Reviews Clinical Oncology, 10, 267-276 (2013); Wang et al. (2012) J. Immunother. 35(9): 689-701; and Brentjetis et al., Sci Transl Med. 2013 5(177). See also WO2014031687, U.S. Pat. No. 8,339,645, U.S. Pat. No. 7,446,179, US 2013/0149337, U.S. Pat. No. 7,446,190, and U.S. Pat. No. 8,389,282.

In another aspect, the target antigens are intracellular. In some embodiments, cells expressing CARs, for example T-cells and NK cells, comprising HLA-A2-restricted epitopes directed to the intracellular target antigens are desirable. Intracellular antigens can include proapoptotic factors/cell death molecules, BRCA1, BRCA2, and the like. Methods of creating CARs that target intracellular antigens have been described in the literature (Tassev et al., Cancer Gene Ther. 2011; Stewart-Jones et al., Proc Natl Acad Sci US A. 2009;106:5784-5788). Other methods are readily apparent to one of skill in the art.

In yet another aspect, the receptor can include anti-fluorescein thiocyanate (FITC)-binding domain that targets FITC-conjugated therapeutic molecules such as monoclonal antibodies, aptamers, ligands etc., that are specific to one or more FITC-labeled target antigens on a breast cell, such as breast cancer cell. Such a method is desirable for targeting multiple target antigens which can limit the ability of a tumor to evade targeting by downregulating a single target antigen. This is also advantageous in tracking the binding of the target antigen on a breast cell and the rate/speed of migration and infiltration of the administered cells within the affected breast tissue such as breast tumor.

Methods of preparing chimeric receptors are known in the art. Exemplary methods of making have been described in US 2016/0206656, US 2016/0045551, US 201/40314795, US 2014/0314795. Methods of preparing RNA CARs have been described by (Barrett et al., Hum. Gene Ther. 2011, 22(12):1575-1586).

Additional CARs and methods of making them have been described: CEA (e.g., colon) (Nolan, et al., Clin Cancer Res., 5:3928-3941 (1999)); EGFRvIII - Bullain, et al., J Neurooncol. (2009), Morgan, et al., Hum Gene Ther., 23:1043-1053 (2012)); ErbB2 (HER2) - Zhao, et al., J Immunol., 183:5563-5574 (2009), Morgan, et al., Mol Ther., 18:843-851 (2010), Pinthus, et al., 114:1774-1781 (2004), Teng, et al., Hum Gene Ther., 15:699-708 (2004), Stancovski, et al., J Immunol., 151 :6577-6582 (1993), Ahmed, et al., Mol Ther., 17:1779-1787 (2009), Ahmed, et al., Clin Cancer Res., 16:474-485 (2010), Moritz, et al., Proc Natl Acad Sci U.S.A., 91 :4318-4322 (1994); ErbB receptor family - Davies, et al., Mol Med., 18:565-576 (2012), Sun et al., Breast Cancer Res.16:R61; ErbB3/4 - Muniappan, et al., Cancer Gene Ther., 7:128-134 (2000), Altenschmidt, et al., Clin Cancer Res., 2:1001-1008 (1996); HLA-A1/MAGE1 - Willemsen, et al., Gene Ther., 8:1601-1608 (2001), Willemsen, et al., J Immunol., 174:7853-7858 (2005)); HLA-A2/NY-ESO-1 - Schuberth, et al., Gene Ther., 20:386-395 (2013); FR-α - Song et al. Blood. 2012 Jan 19;119(3):696-706; Oncoimmunology. 2012 Jul 1; 1(4): 547-549; J. Hematology & Oncology (2016) 9:56; Hwu, et al., J Exp Med., 178:361-366 (1993), Kershaw, et al., Nat Biotechnol., 20:1221-1227 (2002), Kershaw, et al., Clin Cancer Res., 12:6106-6115 (2006), Hwu, et al., Cancer Res, 55:3369-3373 (1995), FAP - Kakarla. et al., Mol Ther. (2013), 21 8, 1611-1620); GD2 - Pule, et al., Nat Med., 14:1264-1270 (2008), Louis, et al., Blood, 118:6050-6056 (2011), Rossig, et al., Int J Cancer., 94:228-236 (2001); IL13R alpha.2 - Kahlon, et al., Cancer Res., 64:9160-9166 (2004), Brown, et al., Clin Cancer Res. (2012), Kong, et al., Clin Cancer Res., 18:5949-5960 (2012), Yaghoubi, et al, Nat Clin Pract Oncol.. 6:53-58 (2009)); Lewis Y - Peinert, et al , Gene Ther.. 17:678-686 (2010), Westwood, et al., Proc Natl Acad Sci U.S.A., 102:19051-19056 (2005), Mezzanzanica, et al, Cancer Gene Ther, 5:401-407 (1998); Mesothelin - Lanitis. et al., Mol Ther., 20:633-643 (2012), Moon, et al, Clin Cancer Res.. 17:4719-4730 (2011); Muel - Wilkie, et al., J Immunol., 180:4901-4909 (2008); NKG2D ligands - Barber, et al, Exp Hematol., 36.1318-1328 (2008), Lehner, et al.. PLoS One, 7:e31210 (2012), Song, et al., Gene Ther., 24:295-305 (2013), Spear, et al., J Immunol., 188:6389-6398 (2012); TAG72 (eg., colon) (Hombach, et al., Gastroenterology, 113 1163-1170 (1997), McGuinness, et al., Hum Gene Ther., 10.165-173 (1999).

### Genetic Engineering - Modified Cells Expressing Recombinant Receptors.

In some embodiments, the cells include one or more nucleic acids introduced by genetic engineering and thereby express recombinant or genetically engineered products, such as recombinant chimeric receptors (e.g., CARs) of such nucleic acids The genetic engineering typically involves the transfer of a nucleic acid encoding the recombinant chimeric receptor into the cell such as by retroviral, lentiviral transduction, transfection, or transformation ((see, e g., Wang et al. (2012), J. Immunother. 35(9): 689-701; Cooper et al. (2003), Blood 101.1637-1644; Verhoeyen et al. (2009), Methods Mol Biol 506. 97-114; and Cavalieri et al (2003), Blood. 102(2). 497-505, Chicaybam et al, (2013). PLoS ONE 8(3). e60298 and Van Tedeloo et al. (2000). Gene Therapy 7(16): 1431-1437). In some embodiments, the cells may first be primed, i.e., stimulated with a stimulus that induces a response such as proliferation, survival or activation. Such response may be measured by expression of an activation marker or cytokine release. Nucleic acid transfer may then be accomplished by transduction, transfection or transformation of the activated cells followed by expansion in cell culture to numbers sufficient for use in clinical applications. Methods for nucleic acid transfer are known in the art.

Nucleic acid transfer into cells may be performed by using recombinant infectious virus particles such as vectors derived from simian virus 40 (SV40), adenovirus, adeno-associated virus (AAV). In some embodiments, the nucleic acid transfer may be done using recombinant lentivial vectors or retroviral vectors such as gamma-retroviral vectors (see, e.g., Koste et al. (2014), Gene Therapy 2014 Apr. 3. doi: 10.1038/gt.2014.25; Carlens et al. (2000), Exp Hematol 28(10): 1137-46; Alonso-Camino et al. (2013), Mol Ther Nucl Acids 2, e93; Park et al., Trends Biotechnol. 201 1 November 29(11): 550-557.

In some embodiments, the retroviruses include those derived from any avian or mammalian cell source. The retroviruses typically are amphotropic, meaning that they are capable of infecting host cells of several species, including humans. In one embodiment, the gene to be expressed replaces the retroviral gag, pol and/or env sequences. A number of illustrative retroviral systems have been described (e.g., U.S. Pat. Nos. 5,219,740; 6,207,453; 5,219,740; Miller and Rosman (1989), BioTechniques 7:980-990; Miller, A. D. (1990), Human Gene Therapy 1:5-14; Scarpa et al. (1991), Virology 180:849-852; Burns et al. (1993), Proc. Natl. Acad. Sci. USA 90:8033-8037; and Boris-Lawrie and Temin (1993), Cur. Opin. Genet. Develop. 3:102-109. Methods of lentiviral transduction are known. Exemplary methods are described in, e.g., Wang et al. (2012), J. Immunother. 35(9): 689-701; Cooper et al. (2003), Blood. 101:1637-1644; Verhoeyen et al. (2009), Methods Mol Biol. 506: 97-114; and Cavalieri et al. (2003), Blood. 102(2): 497-505.

Nucleic acids useful for the present invention include DNA including genomic and cDNA, RNA including mRNA. In some embodiments, expression of the recombinant chimeric receptors may be constitutive or inducible. In other embodiments, the expression of chimeric receptors may be transient, conditionally active or switchable.

Conditionally active CARs may be prepared as described in US20160207989. Inducible CARs may be prepared as described in US20160046700. The inducible chimeric signaling molecules discussed herein allow for a sustained, modulated control of a chimeric antigen receptor (CAR) that is co-expressed in the cell. The inducible chimeric signaling molecules comprise the inducible recombinant receptors (such as CARs) discussed herein. The activation of the target antigen-specific cell such as a T cell, designed to target a cellular antigen implicated in a breast disorder, is dependent on the administration of a ligand inducer. The ligand inducer activates the CAR-expressing cell by multimerizing the inducible chimeric signaling molecules, which, in turn, activates NFₖB signaling and other intracellular signaling, pathways, which activates the cell, for example, a T cell, a tumor-infiltrating lymphocyte, a natural killer cell, or a natural killer T cell. In the absence of the ligand inducer, the modified cells are quiescent, or has a basal level of activity. Dosing of the ligand determines the rate and magnitude of the CAR-expressing cell proliferation and activation.

Transient expression is typically engineered by transiently modifying the cells with RNA CARs (See US20160151491). RNA transgenes can be delivered to a cell and expressed therein following a brief in vitro cell activation, as a minimally expressing cassette without the need of additional viral sequences. The RNA CAR sequence may remain extrachromosomal. In vitro transcribed RNA (IVT-RNA) have been successfully used to modify cells and IVT-RNA vectors are also known in the art (Barrett et al., Hum. Gene Ther. 2011, 22(12):1575-1586). RNA transfer can be done by transfection, electroporation and various other methods known to person of skill in the art. (See, Singh et al., Oncoimmunology 3:12., e962974. December 2014; Schutsky et al., Oncotarget 2015, 6(3))28911-28). IVT-RNA may be stabilized using various modification in order to achieve prolonged expression of the transferred IVT-RNA. IVT vectors are known in the literature which when utilized in a standardized manner for in vitro transcription and which have been genetically modified in such a way that stabilized RNA are produced. mRNA-vaccine technologies are optimized for the production of stable mRNA are useful for the purpose of this invention (Schlake et al., RNA Biol. 2012 Nov 1; 9(11): 1319-1330; Sahin et al., Nature Reviews Drug Discovery 13, 759-780 (2014)).

RNA has several advantages over the more traditional plasmid or viral approaches. *Gene* expression from an RNA source does not require transcription and the protein product is produced rapid after transfection. Since RNA need not enter nucleus and remains in cytoplasm, transfection efficiency is typically high.

CAR expression may be made switchable, i.e., CAR expression may be switched off in a cell when desired or necessary and/or the cells may be eliminated by including in the transferred nucleic acid sequences elements that permit elimination of the CAR expression and of the cells themselves as described below.

Optionally, the nucleic acid sequences may include a tag, such as E-Tag or FITC that would allow the cells to be isolated and purified (PLOS One. 2014, 9(4), e93745). Expression of the recombinant receptor such as a CAR may be confirmed by any suitable method known in the art, for example, by immunohistochemistry, ELISA, FACs, etc.

In an aspect, modified cells may include viruses, for example, by co-transfection with receptors and proteins, such as oncolytic viruses, that aid in their migration to the tumor cells (van Seggelen et al., Mol. Ther. - Oncolytics 2, Article number: 15014 (2015). Oncolytic viruses (OVs), such as Vesicular stomatitis virus, have the capacity to induce specific lysis of tumor cells and indirectly impact tumor growth via vascular shutdown. As modified cells for example, T cells, readily circulate through the body, using these cells to deliver oncolytic viruses directly to tumors provides an ideal combination. Methods of loading oncolytic viruses have been described by van Seggelen (*Id*.). OVs may be loaded into modified cell prior to administering the adoptive cell therapy. Such oncolytic viruses enhance and/or synergize the immune functions of modified cells expressing recombinant receptors such as CARs (Nishio et al., Cancer Res. 2014 Sep 15;74(18):5195-205; Walker et al. 2016. posted online May. 30, 2016; doi: http://dx.doi.org/10.1101/055988). Thus, in some embodiments, the modified cells include oncolytic virus. In another aspect, target antigens include specific chemokine receptors that match relevant tumor-secreted chemokines such as Gro-alpha, CCL17, CCL2. These are advantageous in aiding the modified cells in migrating to the tumor cells. Transpapillary administration close to the tumor sites is advantageous in reducing time and distance for migration *in vivo.*

### Cell Expansion

In some embodiments, cells may be expanded *ex vivo,* and frozen and stored prior to use or administered to a subject immediately. In some embodiments, the modified cells are *ex vivo* expanded prior to transpapillary administration into a breast duct of a subject. In general, cells may expanded by adding the cells to a culture-initiating composition feeder cells, such as non-dividing PBMCs such that the resulting population of cells contain at least 5, 10, 15, 20, 30 or 40 or more PBMC feeder cells for each modified cell such as T-cells, in the initial population to be expanded. Incubation may also be carried out by adding non-dividing EBV-transformed lymphoblastoid cells (LCL) as feeder cells. Culture is incubated at least 25°C, at least 30°C, or generally at 37°C, for time sufficient to expand the modified cells. The feeder cells may be gamma irradiated to prevent cell division.

Methods of *ex vivo* expansion of cells are described in the literature (Terakura et al. 2012, Blood, 119; 72-82; Cartellieri et al., PLoS One, 2014, Vol 9(4), e93745). As a non-limiting example, T-cells expressing recombinant receptors such as CARs are suspended in with gamma-irradiated TM-LCL cells and supplemented with IL-2 and IL-15 every 48 hours. Stimulated CAR's-expressing cells can bifurcate into 2 subpopulations. Cells with high expression of CAR and also expressing CD25 (i.e., CAR+/CD25+) population are desirable (Chang et al., J. of Trans. Med. (2015) 13:161). Stimulated high and low CAR-expressing populations are isolated by FACs after 20 hours of co-incubation with target cells at a particular effector-to-target (E:T) ratio, such as 1:1, or 2:1 etc.

In some embodiments, modified cells are high expressors of recombinant receptors such as CARs. In other embodiments, modified cells may express recombinant receptors, such as CARs, at varying levels (i.e., such cells may be mixtures of high, intermediate and low expressors of the recombinant receptor).

Any chemical synthetic or recombinant mutagenic method may be used to generate a CAR and modified cells expressing a CAR. The practice of the present invention may employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press: 1989); DNA Cloning, Volumes I and II (D. N. Glover, ed., 1985); Oligonucleotide Synthesis (M. J. Gait, ed., 1984); Mullis et al., U.S. Pat. No. 4,683,195; Nucleic Acid Hybridization (B. D. Hames & S. J. Higgins, eds. 1984); Transcription And Translation (B. D. Hames & S. J. Higgins, eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Cabs, eds., 1987, Cold Spring Harbor Laboratory); Methods In Enzymology, Vols. 154 and 155 (Wu et al., eds.), Immunochemical Methods In Cell And Molecular Biology (Mayer and Walker, eds., Academic Press, London, 1987); Handbook Of Experimental Immunology, Volumes 1-IV (D. M. Weir and C. C. Blackwell, eds., 1986); Manipulating the Mouse Embryo (Cold Spring Harbor Laboratory Press, Cold Spring Harbor, N. Y., 1986).

### Safety,

Administration of adoptive cell therapy such as treatment with modified cells expressing chimeric receptors such as CARs can induce severe toxic outcomes or side effects such as cytokine release syndrome (CRS), macrophage activation syndrome (MAS), tumor lysis syndrome (TLS), neurotoxicity, and/or host immune response against the cells and/or recombinant receptors being administered (Bonifant et al. Mol. Ther. - Oncolytics (2016) 3, 16011).

Accordingly, it is desirable to control the expression of chimeric receptors on modified cells (for example, reduce the expression of CARs) or eliminate the modified cells expressing chimeric receptors such as CARs (switchable CAR expression). In some embodiments, the expression of the receptors may be constitutive, inducible, transient, or switchable. In at least one embodiment, the chimeric receptors on modified cells are conditionally active. In some embodiments, the CARs are switchable CARs. In other embodiments, the CARs are inhibitory CARs (iCARs). In still other embodiments, the CARs are self-limiting CARs (in that they are expressed by RNA transfer and their expression is transient).

Thus, the engineered cells may include cellular safety switches, for example, gene segments that cause cells to be susceptible to negative selection *in vivo,* such as upon administration in adoptive cell therapy. For example, in some aspects, the cells are engineered so that they can be eliminated as a result of a change in the *in vivo* condition of a subject to which they are administered. The negative selectable phenotype may result from the insertion of a death genes or suicide gene that confers sensitivity to an administered agent. For example herpes simplex virus thymidine kinase (HSVtk) that mediates the conversion of ganciclovir to ganciclovir triphosphate, which is toxic to dividing cells may be inserted into donor modified cells. Insertion of HSVtk into donor modified cells enables them to be eliminated by the administration of ganciclovir (extracellular switch signal) to a subject. Additional examples death genes or suicide genes include various apoptosis related genes, such as inducible caspase 9 (iC9) and FADD, which may be used to eliminate modified cells in response to chemical inducers of dimerization (CIDs) such as AP20187 (available from ARLAD Pharmaceuticals) and/or AP1903, a small molecule that is safe for dosing human subjects (Sick-Keen Tey. Clin. Translational Immunology (2014) 3, e17; Straathof, et al. Blood, 2005 Jun 1; 105(11): 4247-4254). Such safety switches can also be used for pluripotent stem cell-based therapies (Wu et al. Molecular Therapy - Methods & Clinical Development 1, Article number: 14053 (2014) doi:10.1038/mtm.2014.53).

In still another aspect, a safety switch can be embedded in the extracellular Ag-binding domains of the chimeric receptors expressed by a modified cell that permits switching off activity of the modified cells or eliminating such cells (for example, switchable CAR-T-cells, CAR-NK cells, or CAR-CTLs). Switches can be semi-synthetic switches or fully recombinant switches. Exemplary switches that are responsive to an introduced soluble agent (extracellular switch signal) at an appropriate time include FITC-based semi-synthetic switches and a short peptide neo-epitope (PNE) wholly recombinant intracellular switch. Switches and methods of making and using them have been described (Cao et al., Cancer Immunotherapy, Angew Chem. Int. Ed. 2016, 55, 1-6; Cao et al., Angew. Chem. Int. Ed. 2016, 55, 7520-7524; Rodgers et al., 2016, Proceedings of the National Academy of Sciences of the U.S.A. vol 113(4), E459-E468; Flemming, Alexandra, Nature Reviews Drug Discovery, 15, 15 7 (2016); Ma et al., PNAS. 2016. 113(4): E450-E458), each incorporated by reference herein in its entirety. For example, the FITC and PNE switches have been included in anti-HER2 CARs to study the switchable expression of anti-HER2 CARs (Cao et al., Angew. Chem. Int. Ed. 2016, 55, 7520-7524). Accordingly, the adoptive cell therapy may be switchable. In some embodiments, safety switches include, but are not limited to, death genes or suicide genes such as HSVtk, iC9, and FADD, FITC-based synthetic switches, and PNE switches.

Modified cells expressing a CAR may be self-limiting, for example, the chimeric receptor (CARs) may be transiently expressed in the modified cells. Methods of expressing recombinant proteins transiently are known in the art, for example, transfection with RNA transgenes coding for the chimeric receptors.

Disclosed herein are methods that result in lower degree of toxicity, toxic outcome or symptoms, toxicity-promoting profile, factor or property such as a symptom or outcome associated with or indicative of CRS, MAS, TLS, and neurotoxicity. In some embodiments, the toxic outcome is or associated with CRS. In other embodiments, the toxicity is associated with MAS. In yet other embodiments, the toxicity is neurotoxicity. transpapillary treatment of subjects having or at risk of having breast disorders is desirable for reducing such toxic effects. In some aspects, a lower degree of toxicity, outcome, symptom, profile, factor or property is observed in the subjects to which the compositions disclosed herein are administered transpapillarily.

Exemplary outcomes associated with CRS, MAS, TLS etc., are known in the art and it will be apparent to a person of skill in medicine. CRS, for example, may occur in some cases following adoptive cell therapy and administration of other biological products, and typically CRS typically occurs 6-20 days after infusion of cells expressing CAR (Davila et al., Sci. Transl. Med. 6, 224ra25 (2014); Brentjens et al., Sci. Transl. Med. 5, 177ra38 (2013); Xu et al., Cancer Letters. 343 (2014) 172 -178). Such side effects or outcomes parallel high levels of circulating cytokines such as IFNγ, TNFα, and IL-2 which may underlie observed toxicity. Additional cytokines that are also elevated include but are not limited to, GM-CSF, IL-1beta, IL-6, IL-7, IL-8, IL-10, IL-12, Flt-3, fractalkine, MIP1, sIL-2Rα, and IL-5.

The invention contemplates that cytokine levels of the subject being treated will be monitored and the dose may be adjusted accordingly by the attending physician if cytokine levels increase greater than two-fold. In some embodiments, the first dose includes the cells in an amount that does not cause or reduces the likelihood of toxicity or toxic outcomes such as CRS-related, MAS-related, or TLS-related outcomes, or host immune response against the cells and/or recombinant receptors being administered, fever of at least 38°C for 3 or more days and plasma CRP levels of 20 mg/dL, and/or neurotoxicity. In other embodiments, the dose may depend on the level of side effects or toxic outcomes such as CRS-related, MAS-related, or TLS-related outcomes, and neurotoxicity following initial dose.

CRS may be treated with anti-inflammatory therapy such as anti-IL6 therapy. Such therapy includes anti-IL-6 antibodies such as siltuximab and tocilizumab directed to IL-6 receptor, or antibiotics. In some embodiments, the subject may be treated with such a therapy at any time during the treatment regimen. In at least one embodiment, the subject may be treated with such as therapy following an initial dose. In other embodiment, the subject may be treated with such as therapy following completion of all doses.

CRS, MAS, TLS, neurotoxicity and like observed on adoptive cell therapy may also be treated by switching off the expression of CARs administered cells (and *in vivo* expanded cells) by administering to the subjects CIDs or PNE triggers disclosed herein (or known in the art) and/or eliminating the cells.

### Combination Therapy

The present invention contemplates that transpapillary methods disclosed herein further comprise combination therapy. In an aspect, the transpapillary method further comprises administering to the subject one or more additional therapeutic agent or therapy. The additional therapeutic agents may be administered to the subject by any suitable means known in the art, including without limitation, transpapillarily, orally, nasally, parenterally by injection or infusion, subcutaneously, etc. The additional therapeutic agents may be comprised in the compositions disclosed herein or may be independently formulated. The order of administration of the therapeutic agents and/or therapy may be in any order of administration. For example, the cells or compositions disclosed herein may be co-administered with a therapeutic agent or the therapeutic agent may be administered first or the therapeutic agent may be administered after cells or compositions of the present invention are administered.

The additional therapeutic can be any that is useful for the purpose of the disclosed methods.

Exemplary additional therapeutic agents include, without limitation, asparaginase, busulfan, carboplatin, cisplatin, daunorubicin, doxorubicin, fluorouracil, gemcitabine, hydroxyurea, methotrexate, paclitaxel, rituximab, trastuzumab, vinblastine, vincristine, oncolytic viruses, anti-estrogens such as tamoxifen, endoxifen, N-methyl-endoxifen, norendoxifen, raloxifen, fulvestrant and/or aromatase inhibitors such as anastrozole, letrozole, and exemestane.

Additional therapy may include surgery, radiation, chemotherapy, acupuncture, non-transpapillary adoptive cell therapy, etc. The methods disclosed herein may be used as a primary therapy, neoadjuvant therapy (for example, before surgery (such as mastectomy or lumpectomy) or chemotherapy), or adjuvant therapy (for illustrative purposes only, after chemotherapy or treatment with other methods of adoptive cell therapy,).

### Compositions and Formulations

Also provided herein are compositions comprising modified cells for administration, including pharmaceutical compositions and formulations such as unit dose form compositions, including the number of cells for administration in a given dose or a fraction thereof. The pharmaceutical compositions and formulations generally include one or more optional pharmaceutically acceptable carrier or excipient. In some embodiments, the composition includes at least one additional therapeutic agent. The additional therapeutic agent is selected from the group consisting of asparaginase, busulfan, carboplatin, cisplatin, daunorubicin, doxorubicin, fluorouracil, gemcitabine, hydroxyurea, methotrexate, paclitaxel, rituximab, vinblastine, vincristine, oncolytic viruses, anti-estrogens such as tamoxifen, endoxifen, raloxifen, fulvestrant and/or aromatase inhibitors such as anastrozole, letrozole, and exemestane.

As used herein, the term "pharmaceutically acceptable formulation" refers to a preparation which in such form as to permit the biological activity of an active ingredient contained herein to be effective, and which contains no additional components which are unacceptably toxic to a subject to which the formulation is administered.

As used herein, the term "pharmaceutically acceptable" or "pharmacologically acceptable" means materials, compositions, or vehicles that are compatible with other ingredients of the formulation and that they do not substantially produce adverse reactions, e.g., toxic, allergic, or immunological reactions, when administered to a subject. They may be approved by a regulatory agency, e.g., of the U.S. Federal or state government or listed in the U.S. pharmacopeia or other generally recognized pharmacopeia for use in animals, and more particularly in humans.

As used herein, the term "pharmaceutically acceptable carrier" or "carrier" means a pharmaceutically acceptable material or ingredient which is non-toxic to a subject. A pharmaceutically acceptable carrier includes without limitation, a buffer, excipient, stabilizer, or preservative.

Formulation for transpapillary administration may be any formulation that is suitable for administering into a breast milk duct. For the purpose of transpapillary administration as disclosed herein, the formulation may be a solution, a gel, a suspension, or an emulsion. Carrier to be selected may be determined in part by the particular cell and/or by the method of administration. Carriers are described, e.g., by Remington's Pharmaceutical Sciences, 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as polyethylene glycol (PEG).

Suitable preservatives may include, for example, methylparaben, propylparaben, sodium benzoate, and benzalkonium chloride. In some aspects, a mixture of two or more preservatives is used. The preservative or mixtures thereof are typically present in an amount of about 0.0001% to about 2% by weight of the total composition.

Buffering agents in some aspects are included in the compositions. Suitable buffering agents include, for example, citric acid, sodium citrate, phosphoric acid, potassium phosphate, and various other acids and salts. In some aspects, a mixture of two or more buffering agents is used. The buffering agent or mixtures thereof are typically present in an amount of about 0.001% to about 4% by weight of the total composition. Methods for preparing administrable pharmaceutical compositions are known. Exemplary methods are described in more detail in, for example, Remington: The Science and Practice of Pharmacy, Lippincott Williams & Wilkins; 21st ed. (May 1, 2005).

The formulations can include aqueous solutions. The formulation or composition may also contain more than one active ingredient useful for the particular indication, disease, or condition being treated with the cells, preferably those with activities complementary to the cells, where the respective activities do not adversely affect one another. Such active ingredients are suitably present in combination in amounts that are effective for the purpose intended. Thus, in some embodiments, the pharmaceutical composition further includes other pharmaceutically active agents or drugs, such as chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, trastuzumab, vinblastine, vincristine, oncolytic viruses, anti-estrogens such as tamoxifen, non-endoxifen, endoxifen, raloxifen, fulvestrant and/or aromatase inhibitors such as anastrozole, letrozole, and exemestane.

The pharmaceutical compositions in some embodiments contain the cells in an amount effective to treat or prevent breast disorders, such as therapeutically or prophylactically effective amount. Therapeutic or prophylactic efficacy is monitored in some embodiments, by periodic assessment of the treated subjects.

The desired dosage can be administered transpapillarily in single bolus administration of cells, by multiple bolus administration of cells (split unit dose) or by continuous administration of cells. Administration of cells can be autologous or heterologous/allogeneic. For example, the cells can be obtained from one subject, and administered to the same subject or to a different compatible subject. Peripheral blood derived immune cells or their progenitors (whether *in vivo, ex vivo,* or *in vitro* derived) can be administered transpapillarily.

Formulations to be delivered transpapillarily are provided as sterile liquid preparations (for example, sterilized by filtration through sterile filter membranes), e.g., isotonic aqueous solutions, suspensions, emulsions, dispersions, or viscous compositions including gels, which may be buffered to a selected pH. Liquid preparations are normally easier to prepare than gels and other viscous compositions. Additionally, liquid compositions are somewhat more convenient to administer transpapillarily.

Viscous compositions, including gels, can be formulated within appropriate range of viscosity to provide longer contact periods with specific tissues, such as breast duct lining. Liquid or viscous compositions including gels, can comprise carriers which can be a solvent or dispersion medium containing for example, water, saline, phosphate buffered saline, TRIS-buffered saline, lactated Ringers solution (USP), polyol (for example, glycerol, propylene glycerol, polypropylene glycerol, liquid polypropylene glycol, liquid polyethylene glycol), and suitable mixtures thereof.

Sterile transpapillarily administrable solutions can be prepared by incorporating the cells in a solvent, such as an admixture with a suitable carrier, diluent, or excipient such as sterile water, physiological saline, glucose, dextrose, or the like. The compositions can further include additional substances such as wetting agents, dispersing agent, or emulsifying agents such as methylcellulose, pH buffering agents, gelling or viscosity enhancing agents, preservatives, flavoring gents, colors or any combination thereof.

Gelling agents can be selected from the group consisting of polyacrylic acid, (CARBOPOL^{®}, B.F. Goodrich Specialty Polymers and Chemicals Div. of Cleveland, Ohio), carboxypolymethylene, carboxymethylcellulose and the like, including derivatives of Carbopol^{®} polymers, such as Carbopol^{®} Ultrez 10, Carbopol^{®} 940, Carbopol^{®} 941, Carbopol^{®} 954, Carbopol^{®} 980, Carbopol^{®} 981, Carbopol^{®} ETD 2001, Carbopol^{®} EZ-2 and Carbopol^{®} EZ-3, carboxyvinyl polymers (carbomers), polloxomers, poloxamines, chitosan, dextran, pectins, natural gums, Pemulen^{®} polymeric emulsifiers, and Noveon^{®} polycarbophils, acrylic copolymers such as acrylate/alkylacrylate copolymers, polyacrylamides, cellulose derivatives, ethyl cellulose, hydroxypropyl cellulose, hydroxyethyl cellulose, hydroxypropyl methyl cellulose (HPMC), and carboxymethyl cellulose (CMC), bentones, fatty acid metal salts such as aluminum stearates and hydrophobic silica, or ethylcellulose and polyethylene. Additional thickening agents, enhancers and adjuvants may generally be found in Remington's The Science and Practice of Pharmacy, Meade Publishing Co., United States Pharmacopeia/National Formulary. Hydrophilic gelling agents include polyacrylic acid (carbomer), polysaccharides, such as hydroxypropylcellulose, natural gums and clays, and, as lipophilic gelling agents, representative are the modified clays. In some embodiment, the gelling agent is HPMC. In other embodiments, the gelling agent is CMC. In still other embodiments, the gelling agent is Carbopol. In yet another embodiment, the gelling agent is alginate or gelatin.

The concentration of gelling agent can be adjusted to change the viscosity of the gel. For example, in some embodiments, the formulation includes less than 0.1%, 0.5%, 1%, less than 2% less than 3%, less than 4%, less than 5%, less than 10% of the gelling agent. Alternatively, the gelling agent can be in the range of 0.1% to 80% w/w of the composition.

Various additives which enhance the stability and sterility of compositions, including microbial preservatives, antioxidants, chelating agents, and buffers can be added. Antimicrobials, such as various antibacterials and antifungals, for example, parabens, chlorobutanol, phenol, and sorbic acid can be incorporated.

Prolonged absorption of the transpapillary pharmaceutical form can be brought about by the use of agents delaying absorption, for example, aluminum monostearate, polyethylene glycol and gelatin.

In another aspect, the compositions comprise gadolinium chelates, superparamagnetic iron oxide nanoparticles (SPION), ¹⁹F perfluorocarbon nanoparticles, and other magnetic reporter genes, such as metalloprotein-based MRI probes.

### Articles of Manufacture

Also provided herein are articles of manufacture such as kits and devices, for the administration of the cells and compositions disclosed herein for adoptive cell therapy, and for storage and administration of the cells and compositions.

The articles of manufacture include one or more containers, typically one or more containers, packaging material, and a label or package insert generally including instructions for administration of the cells to a subject.

The containers contain one or more unit doses of the cells and compositions to be administered. In some embodiments, the article of manufacture comprises one or more containers, each containing a single unit dose of the cells. The unit dose may be an amount or number of the cells to be administered to the subject in the first dose or twice the number (or more) the cells to be administered in the first or subsequent dose(s). It may be the lowest dose or lowest possible dose of the cells that would be administered to the subject in connection with the administration method.

In some embodiments, the unit dose is the minimum number of cells or number of cells that would be administered in a single dose to any subject according to the methods herein. In some embodiments, the number of cells in the unit dose is the number of cells or number of recombinant receptor-expressing or CAR-expressing cells that it is desired to administer to a particular subject in a first dose, such as a subject from which the cells have been derived.

In some embodiments, the cells have been derived from the subject to be treated by methods as provided herein or in need thereof.

In some embodiments, one or more of the unit doses contains cells that express the same receptor, e.g., CAR. In some aspects, one or more of the unit doses contains cells that express a different receptor, e.g., CAR, than one or more of the other unit doses.

In some embodiments, each of the containers individually comprises a unit dose of the cells that express the first, or second, or third, and so forth, receptor, which contains the same or substantially the same number of cells. Thus in some embodiments, each of the containers comprises the same or approximately or substantially the same number of cells or number of recombinant receptor-expressing cells. In some embodiments, the unit dose includes less than 1×10³, less than 0.5×10⁴, less than 1×10⁴, less than 0.5×10⁵, less than 1×10⁵, less than 0.5×10⁶, less than 1×10⁶, less than 0.5×10⁷, less than 1×10⁷, less than 0.5×10⁸, less than 1×10⁸, less than 5X108 modified cells, total cells, T-cells, NK cells, CTLs, macrophages, monocytes, granulocytes, PBMCs, or progenitors thereof.

In some embodiments, the articles of manufacture further include one or more additional other pharmaceutically active agents or drugs, such as chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, trastuzumab, vinblastine, vincristine, oncolytic viruses, anti-estrogens such as tamoxifen, endoxifen, raloxifen, fulvestrant and/or aromatase inhibitors such as anastrozole, letrozole, and exemestane.

Suitable containers include, without limitation, for example, bottles, vials, syringes, and flexible bags, such as infusion bags. The containers may be formed from a variety of materials such as glass or plastic. In some embodiments, the container has one or more port, e.g., sterile access ports, for example, for connection of tubing or cannulation to one or more tubes, e.g., for transpapillary delivery and/or for connection for purposes of transfer to and from other containers, such as cell culture and/or storage bags or other containers.

The article of manufacture may further include a package insert or label with one or more pieces of identifying information and/or instructions for use. In some embodiments, the information or instructions indicates that the contents can or should be used to treat a breast disorder and/or providing instructions therefor. The label or package insert may indicate that the contents of the article of manufacture are to be used for treating the breast disorder. In some embodiments, the label or package insert provides instructions to treat a subject, e.g., the subject from which the cells have been derived, via a method involving the administration of a first and one or more subsequent doses of the cells, e.g., according to any of the embodiments of the provided methods. In some embodiments, the instructions specify administration, in a first dose, of one unit dose, e.g., the contents of a single individual container in the article of manufacture, followed by one or more subsequent doses at a specified time point or within a specified time window and/or after the detection of the presence or absence or amount or degree of one or more factors or outcomes in the subject.

As used herein, the terms "a," "an," and "the" include plural reference unless the context dictates otherwise.

As used herein, the term "about" refers to a measurable value such as an amount, a temporal duration and the like, is meant to encompass variations of ± 20% or in some instances ± 10%, or in some instances ± 5%, in some instances ±1%, and in some instances ±0.1% from the specified value, as such variations are appropriate to perform the disclosed methods.

"Activation" as used herein refers to the state of a cell that has been sufficiently stimulated to induce detectable cellular proliferation. Activation can also be associated with induced cytokine production, and detectable effector functions. The term "activated T cells" refers to, among other things, T cells that are undergoing cell division.

As used herein, "adjuvant therapy" refers to a therapy that follows a primary therapy and that is administered to subjects at risk of relapsing. These are subjects who have a history of breast disorder and have been treated with another mode of therapy. Adjuvant systemic therapy in case of breast cancer usually begins soon after primary therapy to delay recurrence, prolong survival or cure a subject. As used herein "primary therapy" refers to a first line of treatment upon initial diagnosis of a breast disorder in a subject. Non-limiting exemplary primary therapies may involve surgery, a wide range of chemotherapies, and radiotherapy.

As used herein, the term "at risk of having" includes a risk of developing a breast disorder as well as a risk of recurrence of breast disorder.

As used herein, the terms "subject," "patient," and "individual," may be used interchangeably herein and refer to a mammal such as a human. Mammals also include pet animals such as dogs, cats, laboratory animals, such as rats, mice, and farm animals such as cows and horses. Unless otherwise specified, a mammal may be of any gender or sex.

As used herein, a "route of administration" or "route of delivery" for the adoptive cell therapy or compositions of present disclosure refers to the pathway for delivering the cells or compositions of the present disclosure to a subject.

### Specific Embodiments

1. A transpapillary method of adoptive cell therapy for treatment of a subject having or at risk of having a breast disorder comprising administering cells into a breast duct of the subject.
2. The method of Claim 1, wherein the cells comprise a T-cell, an NK cell, a CTL, a TIL, a monocyte, a granulocyte, or progenitors thereof.
3. The method of Claim 1, wherein the cells comprise modified cells expressing one or more recombinant receptors that binds a target antigen on a breast cell.
4. The method of Claim 3, wherein the one or more recombinant receptors comprises a chimeric antigen receptor (CAR) or an engineered or disease-specific TCR.
5. The method of Claim 3, wherein the modified cells express two or more CARs.
6. The method of any one of Claims 3 to 5, wherein the one or more recombinant receptors are independently monospecific, bispecific or multispecific.
7. The method of any one of Claims 3 to 6, wherein the one or more recombinant receptors is/are constitutively, transiently or switchably expressed, or conditionally active.
8. The method any one of Claims 3 to 7, wherein the modified cells comprise a safety switch selected from the group consisting of death gene switches, a FITC-based switches and a PNE-based switches.
9. The method of Claim 8, wherein the death gene switch is a HSV-tk, an iCaspase9 or a FADD.
10. The method of any one of Claims 3 to 9, wherein the target antigen is a tumor specific antigen, a tumor associated antigen, a multi-lineage tumor associated antigen, an oncofetal antigen, a neoantigen, or an immunosuppressive antigen.
11. The method of any one of Claims 3 to 10, wherein the target antigen is selected from the group consisting of transformation-related molecules such as MUCs such as MUC1, c-met, cytokeratins such as CK5, CK6, CK14, CK7, CK8, CK14, CK17, CK18, CK19, p53, glycosides, Tn, TF, and sialyl Tn (STn), Lewis x, Lewis a, Lewis y, and gangliosides such as GM3, GD3, 9-0-acetyl GD3, 9-0-acetyl GT3, and N-glycoly-GM3, Folate Receptor alpha, ROR1, neoantigens, tumor-specific antigens and oncofetal antigens, tumor associated antigens such as carcinoembryonic antigen (CEA), L1 cell adhesion molecule (L1CAM), CAFs-related proteins such as fibroblast activation protein (FAP), FAP-α, FSP-1/S100A4, and PDGFR-β, diganglioside GD2, mesothelin, IL-13 receptor IL13R, IL-13 receptor α, ephrinB2, IGFR1, ELIGHT, WT1, TAG-72, Ep-CAM, LFA-1, EGFR, estrogen receptor (ER), progesterone receptor, MAGE1, MAGE-3, MAGE-A3/6, MAGE-A family members such as MAGE-A1, MAGE-A2, MAGE-A3, MAGE-A4, MAGE-A6, MAGE-A12, MAGE-A9, MAGE-A11, MAGE-C1, and MAGE-C2, RAGE, BCR-ABL, protein tyrosine kinases such as PRL-2 and PRL3, tumor associated glycoproteins such as TAG-72, CA 19-9, CA 27.29, CA 72-4, CA 50, PD-1, CTLA-4, CD47, receptor tyrosine kinases such as H4-RET, Ki-67, cyclin D1, cyclin A, cyclin E, p16, p21, p27, p53, Bcl-2, Bax, survivin, c-myc, Rb, VEGF, HPR1, HER1, HER2, HER3, HER4, CD10, SPARC, COX-2, basal cytokeratins, CK5/6, CK14, and CK 17, epidermal growth factor receptor, c-kit, c-erbB-2, IL-10, TGF-beta, CCL17, CCL22, and CCL24 stroma released factors such as EGF, HGF, MCP-1, CSF-1, VEGF, cytokines such as IL1, IL-8, TNF-alpha, enzymes such as MM2, MMP7, MMP8, MMP9, MMP12, MMP13, and COX2.
12. The method of any one of Claims 3 to 11, wherein the recombinant receptor is a HER2-CAR, FR-α-CAR, or a FAP-CAR.
13. The method of any one of Claims 3 to 12, wherein the recombinant receptor comprises a primary signaling molecule selected from the group consisting of TCR zeta, FcR gamma, FcR beta, CD3 gamma, CD3 delta, CD3 epsilon, CD4, CD8, CD16, CD22, CD25, CD79a, CD79b, and CD66d.
14. The method of any one of Claims 3 to 13, wherein the recombinant receptor comprises one or more co-stimulatory molecules.
15. The method of any one of Claims 3 to 14, wherein the co-stimulatory molecule is selected from the group consisting of MHC class I molecule, BTLA and a Toll ligand receptor, immunoglobulin superfamily (IgSF) such as CD28, B7 receptor family members (B7-H2/B7RP-1/LICOS/GL50, B7-DC/PD-L2, 137-H3), CD226, TIM, CD2/SLAM, BTN, LAIR, tumor necrosis factor receptor superfamily (TNFRSF) such as OX40, CD27, CD30, DR3, GITR, and HVEM, CD2 and SLAM on T-cells, ICAM-1, LFA-1 (CD11a/CD18), adhesion molecules (CD54, CD58, CD70), ICOS, CD40, CD40L, 4-1BB (CD137), CD70, CD80, CD86, DAP10, and other orphan receptor families such as LAG3 (CD223) and CD160.
16. The method of any one of Claims 3 to 14, wherein the recombinant receptor comprises CD28 and 4-1BB as co-stimulatory molecules.
17. The method of any one of Claims 3 to 16, wherein the modified cells comprise a T-cell, an NK cell, a CTL, a monocyte, a granulocyte, or progenitors thereof.
18. The method of any one of Claims 3 to 17, wherein the modified cells further comprise a dye or a contrasting agent selected from the groups consisting of gadolinium chelates, superparamagnetic iron oxide nanoparticles (SPION), ¹⁹F perfluorocarbon nanoparticles, and other magnetic reporter genes, such as metalloprotein-based MRI probes.
19. The method of any one of the preceding claims, wherein the cells are formulated in a composition further comprising a pharmaceutically acceptable carrier, buffer, an excipient or a combination thereof.
20. The method of Claim 19, wherein the carrier is lactated Ringers Solution.
21. The method of Claim 19, wherein the composition further comprises a gelling agent.
22. The method according to any one of the preceding claims, wherein the breast disorder is benign breast disease, breast cancer, Paget's disease of the nipple, or phyllodes tumor.
23. The method according to any one of the preceding claims, wherein the breast disorder is hyperplasia, atypia, ductal hyperplasia, lobular hyperplasia, atypical ductal hyperplasia (ADH), or atypical lobular hyperplasia (ALH).
24. The method according to any one of the preceding claims, wherein the breast disorder is a breast cancer selected from the group consisting of ductal carcinoma in situ (DCIS), lobular carcinoma in situ (LCIS), invasive (or infiltrating) lobular carcinoma (ILC), invasive (or infiltrating) ductal carcinoma (IDC), microinvasive breast carcinoma (MIC), inflammatory breast cancer, ER-positive (ER+) breast cancer, progesterone receptor positive (PR+) breast cancer, ER+/PR+ breast cancer, ER-negative (ER-) breast cancer, HER2+ breast cancer, triple negative breast cancer (i.e., ER-/PR-/Her2-breast cancer; "TNBC"), adenoid cystic (adenocystic) carcinoma, low-grade adenosquamatous carcinoma, medullary carcinoma, mucinous (or colloid) carcinoma, papillary carcinoma, tubular carcinoma, metaplastic carcinoma, or micropapillary carcinoma.
25. The method according to any one of the preceding claims, wherein the breast cancer is a pre-cancer, an early stage cancer, a non-metastatic cancer, a pre-metastatic cancer, a locally advanced cancer, a metastatic cancer or a recurrent cancer.
26. The method according to any one of the preceding claims, wherein the cells are administered in a single dose or multiple doses.
27. The method of Claim 26, wherein the multiple doses comprise a first dose and one or more subsequent doses.
28. The method of Claim 26, wherein one or more dose is administered in a split unit dose.
29. The method according to any one of the preceding claims, wherein the subject is administered a unit dose of cells ranging from 1×10³ to 1×10⁹ modified cells/kg body weight, from 1×10³ to 5×10⁸ modified cells/kg body weight, from 0.5×10³ to 1×10⁷ modified cells/kg body weight, from 1×10⁴ to 0.5×10⁶ modified cells/kg body weight, from 0.5×10⁴ to 1×10⁶ modified cells/kg body weight, from 1×10⁵ to 0.5×10⁶ modified cells/kg body weight.
30. The method of claim 27, wherein the first dose is a low dose, such as less than 1×10³ cells/kg, less than 0.5×10⁴ cells/kg, less than 1×10⁴ cells/kg, less than 0.5×10⁵ cells/kg, less than 1×10³ cells/kg, less than 0.5×10⁶ cells/kg, or less than 1×10⁶ cells/kg.
31. The method of claim 27, wherein the first dose is a high dose, such as greater than 0.5×10⁵ cells/kg, greater than 1×10⁵ cells/kg, greater than 0.5×10⁶ cells/kg, greater than 1×10⁶ cells/kg, greater than 0.5×10⁷ cells/kg, greater than 1×10⁷ cells/kg, greater than 0.5×10⁸ cells/kg, greater than 1×10⁸ cells/kg, or greater than 5×10⁸ cells/kg.
32. The method of Claim 27, wherein a subsequent dose is administered between 7 and 28 days after the initiation of the first dose.
33. The method of Claim 27, wherein a subsequent dose is same as the first dose, lower than the first dose or higher than the first dose.
34. The method according to any one of the preceding claims, wherein the cells are administered as primary therapy, neoadjuvant therapy, or adjuvant therapy.
35. The method according to any one of the preceding claims, wherein the administration of cells reduces disease burden of the breast disorder in the subject.
36. The method according to any one of the preceding claims, the administration of modified cells comprising a recombinant receptor reduces tumor burden in a subject
37. The method according to any one of the preceding claims wherein administration of cells reduces tumor burden.
38. The method according to any one of the preceding claims wherein administration of modified cells reduces tumor burden.
39. The method according to any one of the preceding claims wherein administration reduces a risk of a CRS-related, a MAS-related, a TLS-related, a neurotoxicity-related or a host immune response-related outcome.
40. The method according to any one of the preceding claims, wherein the administration of cells reduces circulating or breast tissue levels of cytokines such as IFNy, TNFα, IL-2, GM-CSF, IL-1beta, IL-6, IL-7, IL-8, IL-10, IL-12, Flt-3, fractalkine, MIP1, sIL-2Rα, and IL-5.
41. The method according to any one of the preceding claims, wherein the administration of modified cells reduces circulating or breast tissue levels of cytokines such as IFNγ, TNFα, IL-2, GM-CSF, IL-1beta, IL-6, IL-7, IL-8, IL-10, IL-12, Flt-3, fractalkine, MIP1, sIL-2Rα, and IL-5.
42. The method according to any one of the preceding claims, wherein the subject is preconditioned with a lymphodepleting agent or a chemotherapeutic agent prior to administration of the cells.
43. The method according to Claim 42, wherein the lymphodepleting agent or a chemotherapeutic agent is selected from the group consisting of cyclophosphamide, cyclosporine, fludarabine, bendamustine, lenalidomide, pomalidomide, gemcitabine, BTK inhibitors such as ibrutinib, oncolytic adenovirus or combinations thereof.
44. The method of any one of the preceding claims, wherein the subject is administered an additional therapeutic agent or therapy.
45. The method of Claim 44, wherein the additional therapeutic agent is selected from the group consisting of asparaginase, busulfan, carboplatin, cisplatin, daunorubicin, doxorubicin, fluorouracil, gemcitabine, hydroxyurea, methotrexate, paclitaxel, rituximab, vinblastine, vincristine, oncolytic viruses such as oncolytic adenovirus, anti-estrogens such as tamoxifen, N-methyl-endoxifen, nor-endoxifen, endoxifen, raloxifen, fulvestrant and/or aromatase inhibitors such as anastrozole, letrozole, and exemestane.
46. A transpapillary method of adoptive cell therapy for treatment of a subject having or at risk of having a breast disorder comprising administering modified cells expressing a HER2-CAR, a FAP-CAR, or a FR-α into a breast duct of the subject.
47. The method of Claim 46, wherein the HER2-CAR has at least 80%, at least 81%, at least 82%, at least 83%, at least 84%, at least 85%, at least 86%, at least 87%, at least 88%, at least 89%, at least 90%, at least 91%, at least 92%, at least 93%, at least 93%, at least 94%, at least 95%, at least 96%, at least 97%, at least 98%, at least 99%, and 100% homology to SEQ ID NO:1 disclosed in FIGURE 2 or a variant or functional portion thereof.
48. The method of Claim 46, wherein the FAP-CAR has at least 80%, at least 81%, at least 82%, at least 83%, at least 84%, at least 85%, at least 86%, at least 87%, at least 88%, at least 89%, at least 90%, at least 91%, at least 92%, at least 93%, at least 93%, at least 94%, at least 95%, at least 96%, at least 97%, at least 98%, at least 99%, and 100% homology to SEQ ID NO:2 disclosed in FIGURE 2 or a variant or functional portion thereof.
49. An article of manufacture, comprising one or more containers, packaging material, a label or package insert, and optionally, a device.
50. The article of manufacture of Claim 49, wherein the device is a needle and syringe, a cannula, a catheter, a microcatheter, an osmotic pump, or an encapsulation device.

Throughout this disclosure, various aspects of the claimed subject matter are presented in a range format or in absolute value or parameter. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range. Where there is a reference to an absolute value or parameter, it is intended that the absolute value or parameter includes the usual error range for the respective value or parameter readily known to the skilled person in this technical field. Reference to an absolute value or parameter herein includes (and describes) embodiments that are directed to that value or parameter per se.

All publications, including patent documents, scientific articles and databases, referred to in this application are incorporated by reference in their entireties for all purposes to the same extent as if each individual publication were individually incorporated by reference. If a definition set forth herein is contrary to or otherwise inconsistent with a definition set forth in the patents, applications, published applications and other publications that are herein incorporated by reference, the definition set forth herein prevails over the definition that is incorporated herein by reference.

The section headings used herein are for organizational purposes only and are not to be construed as limiting the subject matter described.

### Examples

The examples provided herein in the present disclosure are intended as illustrations only and are not intended to limit the scope of the invention.

### Example 1

### Kinetics of Modified Cell Trafficking following Transpapillary Adoptive Cell Therapy

Eight (8) subjects scheduled to undergo surgery for the treatment of breast cancer are enrolled to study the kinetics of modified cells migrating and transiting out of the breast following transpapillary adoptive cell therapy as follows:
**Control Cells.** Modified T-cells are mock transduced with a vector cassette that includes a tag such as Strep-tag II or an anti-flurescein-scFV (Control Cells). Control Cells at doses ranging from 1× 10² to 5×10⁹ are transpapillarily administered into a breast duct of four (4) subject. Blood samples are obtained from the subject at hours zero (0), 1, 2, 4, 8, 12, 24, 36, 48, 72, and 7 days following transpapillary administration of Control Cells into the breast duct(s). Peripheral blood mononuclear cells (PBMCs) are isolated using Lymphocyte Separation Medium (Mediatech Inc, Manassas, VA) according to manufacturer's instructions. Isolated peripheral blood mononuclear cells (PBMCs) were incubated with 10 µl of a 1.2% human IgG solution (Baxter, Deerfield, IL) to block non-specific antibody binding. The PBMCs are cell-sorted by FACS and Control Cells are separated and enumerated. The number and timing of appearance of Control Cells in the peripheral circulation is determined.

**Modified cells expressing recombinant receptors such as CAR-T cells:** HER2-CAR expressing T-cells at doses ranging from 1× 10² to 5×10⁹ are transpapillarily administered into a breast duct of four (4) subject to determine the kinetics of migration and appearance in the peripheral circulation. The modified cells are anticipated to show a delayed appearance in blood and in reduced numbers compared to Control Cells.

Control Cells and HER2-expressing modified cells administered to some subjects are also loaded with gadolinium chelates. Subjects dosed with gadolinium chelate loaded Control Cells and HER2-expressing modified cells undergo MRI scan on day 0 and day 7 to track the migration of the transpapillarily administered cells.

Following the scheduled definitive surgery (either lumpectomy or mastectomy as recommended by the attending physician), breast tissue samples are harvested. ELISA using HER2 is performed on breast tissue samples and T-cell phenotype and local cellular responses such as cytokine release are determined. Sentinel or other lymph nodes are optionally harvested and tested for migration of the administered cells into the tissue samples. It is anticipated that the T-cells have expanded *in vivo,* taken on a memory phenotype and/or has an antitumor cytotoxic activity.

### Example 2

### Treatment of Local Breast Cancer with HER2-CAR-Expressing Lymphocytes

**Clinical Trial:** A Phase I/II clinical trial is conducted with subject having local breast cancer that expresses HER-2 using lymphodepleting pre-conditioning followed by intraductal administration of Anti-HER-2 Gene transduced lymphocytes (modified cells).

Subject inclusion criteria includes breast cancer that expressed ErbB2 (HER-2) at ≥2⁺ as assessed by immunohistochemistry in a Clinical Laboratory Improvement Amendments (CLIA) approved laboratory and for whom a mastectomy following intraductal administration of modified cells are performed. Subjects are ≥ 18 years of age. Declaration of Helsinki protocols are followed and written informed consent from subjects will be obtained.

Before receiving treatment with modified cells (transduced peripheral blood lymphocytes (PBLs)), subjects are transiently lymphoablated using a non-myeloablative lymphodepleting regimen by intravenous administration of cyclophosphamide at 60 mg/kg for 2 days followed by fludarabine at 25 mg/m2 for 5 days. One day after completion of their lymphodepleting regimen, subjects receive modified cells administered intraductally using a catheter followed by high-dose (720,000 U/kg) IL-2 (Aldesleukin; Chiron, Emeryville, CA) every 8 hours to induce tolerance. The protocol is designed as a cell dose escalation in cohorts of three patients each. The lowest cell dose cohort is ≤10³ cells/intraductal administration. The highest dose is 5 × 10⁹.

**Gene transfer procedure:** A γ-retroviral vector plasmid construct designed to express the HER2-specific single-chain Fv fragment (4D5-CD8-28BBZ) which is based on Herceptin mAb is prepared as described in the literature (Zhao Y, et al., J Immunol. 2009;183:5563-5574 and its supplement S1). In brief, the single-chain Fv fragment from mAb 4D5 is linked to the CD8α-chain hinge and transmembrane region with CD28, 4-1BB, and CD3zeta intracellular signaling domains. This cassette is inserted into an MSGV-1 γ-retroviral vector.

A high-titer PG13 cell-based producer cell line is selected and transduced using current good manufacturing practices, and retroviral vector supernatants are collected. The vector supernatants are tested and are in accordance with currently required US Food and Drug Administration guidelines for the production of recombinant γ-retroviral vectors for clinical application.

**The transduction procedure:** P13MCs are stimulated with anti-CD3 mAb OKT3 (Ortho Diagnostic Systems, Raritan, NJ) at a final concentration of 50 ng/ml with recombinant human IL-2 at a final concentration of 300 IU/ml in AIM-V medium (Invitrogen, Carlsbad, CA) containing 5% human serum (Surgery Branch, NCI). Cells are harvested for retroviral transduction on day 2 and resuspended in the same medium without OKT3. Retroviral vector supernatant are thawed and diluted with two parts of medium before being loaded onto RetroNectin (CH-296; Takara Bio, Ohtsu, Japan) coated (coated using 10 mg/ml of CH-296) non-tissue culture treated six-well plates. Vector supernatant are "spun loaded" onto coated plates by centrifugation at 2,000 g for 2 hours at <32 °C. Retroviral vector are aspirated from the wells and 1×10⁶ to 2×10⁶ activated PBMC are added pre-well followed by centrifugation at 1,000g for 10 minutes. Plates are incubated at 37 °C overnight, and the next day all wells are harvested, pooled, and the transduction procedure will be repeated. Following the second transduction, cells are collected and maintained in medium at 0.5 ×10⁶ to 2.0 ×10⁶ cells/ml for a total of 10 days after stimulation. At day 10 after stimulation, cells are subject to a rapid expansion procedure for an additional 14 days using 6,000 IU/ml IL-2 with 50 ng/ml anti-CD3 mAb OKT3 and 100-fold excess 5 Gy irradiated allogeneic PBL feeder cells. Treatment cells are washed in saline before infusion and resuspended in 125 ml containing 300 IU/ml IL-2, and then are administered to the patient intraductally.

**In vitro assays:** Two to four days before infusion, CAR-transduced PBLs are evaluated for ERBB2-specific CAR expression using an ERBB2-Fc fusion protein, or as control, VEGFR2-Fc (R&D Systems, Minneapolis, MN) followed by phycoerythrin-conjugated antihuman IgG Fc antibody (eBioscience, San Diego, CA). Immunofluorescence are analyzed as the relative log fluorescence of live cells, and measured using a FACSCalibur flow cytometer (Becton Dickinson, Franklin Lakes, NJ). Cell function are evaluated by overnight co-culture with HER2-expressing and nonexpressing target cells (1×10⁵ target plus 1 ×10⁵ effector T cells) followed by enzyme-linked immunosorbent assay (ELISA) measurement (Pierce Endogen, Rockford, IL) of IFN-γ. HER2+ target cells such as melanoma cell lines 526, 624, 888, 938 (generated at the Surgery Branch, NCI) and tumor lines, SK-OV3, SK-BR3, MDA361 (American Type Culture Collection, Rockville, MD), and HER2 - tumor lines MDA468 and CCRF-CEM (CEM) obtained from ATCC. All tumor cell lines are cultured in media consisting of RPMI-1640 supplemented with 10% heat inactivated fetal bovine serum (Biofluids, Rockville, MD), 100 U/ml penicillin, 100 µg/ml streptomycin (Invitrogen). Non-transformed human cell cultures, commercially available from Lonza (Walkersville, MD), are purchased and maintained in supplier-recommended media. Autologous dendritic cells and macrophage cultures are obtained from subject's PBMC (Lassus H et al., J. Mol. Med. 2006;84: 671-681).

Genomic DNA is isolated from flash-frozen tissue samples using Maxwell 16 Tissue DNA Purification kit (Promega, Madison, WI) according to the manufacture's instruction. One hundred nanograms of each DNA is used for the Real-time quantitative-PCR assay (TaqMan; Applied Biosystems, Foster City, CA). All PCR are performed using an ABI 7500 Fast Real-time PCR System instrument (Applied Biosystems). The TaqMan gene-specific assay is designed by ABI Assays-by-Designs software (Applied Biosystems). Primers and probe used for detection of the HER2-CAR vector are: 4D51313CD3Z-F TGCCGATTTCCAGAAGAAGAAGAAG (SEQ ID NO: 9), 4D5BBCD3Z-R TGCGCTCCTGCTGAACT (SEQ ID NO: 10), 4D5BBCD3Z-M FAM probe CACTCTCAGTTCACATCCT (SEQ ID NO: 11). The reference standard curve will be established using the DNA extracted from the cells infused into the subject, with undiluted infusion DNA being given a value of 100 as reference. TaqMan β-actin control reagents kit (Applied Biosystems) is used to normalize reactions to input DNA amounts. Cytokine genotype is determined using a commercially available PCR-sequence specific primer kit (Cytokine Genotype Tray; One Lambda, Canoga Park, CA) as directed by the supplier.

Serum cytokine levels will be assayed for using commercially available enzyme-linked immunosorbent assay kits [IFN-γ, TNF-α, GM-CSF, and IL-6 (Endogen, Cambridge, MA)] or Searchlight cytokine array (Aushon Biosystems, Billerica, MA). Cytokine secretion will be measured in samples diluted to be in the linear range of the assay.

### Intraductal Administration of the Modified Cells.

**Nipple Preparation.** After subjects have disrobed, a clinician cleanses the nipple on the breast to be studied. This includes wiping the nipple clean with a slightly granular gel or ointment to loosen and remove any dead skin cells and accumulated oils. This is a cleanser frequently used in hospitals before medical procedures. Afterwards, some numbing cream is applied to the nipple.

**Nipple Anesthetic.** 1 mL of Lidocaine mixed with 0.1-0.2 mL of blue dye is injected with a very small needle into the base of the nipple.

**Duct Identification.** After dye injection and before the catheter placement, a small, flexible wire is inserted about ½ inch into the opening to further identify and dilate the duct opening. Once a duct is identified, a small piece of knotted suture material is inserted into the duct to mark it. This is done on at least 3 duct openings and as many as 5. If the clinician is unable to find at least 3 duct openings, the subject is not able to continue in the study and is withdrawn. These subjects are replaced by newly enrolled subj ects.

**Catheter Placement, Instillation of Transfected T-cells, and X-ray Examination.** Once all of the nipple duct openings are marked, the clinician will insert and place a catheter via the ductal orifice into each marked breast duct so marked. Once the catheters are in place, the clinician may optionally slowly (over 30 seconds) instills less than 1 mL of radio-opaque dye into the ducts to permit imaging of the ducts.

After dye instillation and depending on the number of ducts identified, up to 2 mL (generally ranging from 0.5 to 2 mL) of a suspension of transfected cells is slowly (over 1 minute) injected into each duct. Only the breast containing the invasive cancer is treated. Cells are transpapillarily administered into affected breast ducts in volumes upto10 mL in batches or sets depending on the number of ducts to be treated. Attempts are made to distribute the doses evenly duct-by-duct based upon the number of affected ducts or lobules identified.

The catheter generally remains in each duct for approximately 1-5 minutes. During the procedure, subjects are asked to assess their pain using a visual analog scale. After dye and cells have been instilled an image is taken to demonstrate adequate infusion of modified cells into the ducts. When the number of ducts affected is higher than 2 ducts, the transpapillary adoptive cell therapy procedure may be in done in sets. The catheters are removed from the first set of ducts, for example 2 adjacent duct, and these ducts are each marked with a small piece of knotted suture material. At this point, subjects are assessed for pain using the pain scale for pain assessment.

Subsequently, new catheters are inserted into the remaining marked ducts. After the next half of the nipple ducts have been cannulated, and dye and cells are infused, another image is taken with the fluoroscope to document the ducts. Subjects are asked again to assess their pain. The catheters are removed and ducts individually marked with a small piece of knotted suture material. Benzoin ointment and a clear plastic dressing (bio-occlusive) are placed on the nipple to keep the markers in place until surgery. The total procedure takes 0.5 to 1.5 hours. Photographs are taken of the procedure.

If during assessment of pain, the subject reports Grade 3 or 4 pain in the breast which does not resolve within 10 minutes after infusion of the modified cells, study related procedures are discontinued for that subject. Blood draws and follow-up assessment as well as pathological assessment as described herein are performed per the protocol. In this case, subjects are replaced in the study group for statistical purposes.

If on initial X-ray examination perforation is noted side effects are assessed immediately. If the subject does not report, any untoward effects, the remaining ducts are cannulated and administered with modified cells. Study related blood draws and assessment as well as pathological assessment as described below are performed per protocol.

### Example 3

### Treatment of Ductal Carcinoma in Situ with HER2-CAR-expressing autologous T-cells

The study is designed to determine the safety and feasibility of transpapillary administration of autologous T-cells that have genetic material transferred into the cells to redirect them to target breast cancer cells rather than their usual target. Eligible subjects have HER2+ and/or recurrent breast cancer resistant to one standard therapy or has newly diagnosed HER2+ breast cancer.

Fifteen evaluable patients over the age of 18 years with a baseline ECOG status of 0 or 1 are enrolled in stepwise fashion. Step 1 enrolls subjects with HER2+ and/or recurrent breast cancer resistant to at least one standard therapy, such as tamoxifen or other chemotherapy, and step 2 enrolls subjects in newly diagnosed DCIS. Subjects receive modified T-cells expressing chimeric antigen receptor (iC9-Her2scFV-CD8a hinge-CD28 Tm-CD28 costimulatory domain and CD3ζ intracellular signaling domain) that includes a suicide safety switch, inducible caspase 9 (iC9), and that specifically binds to HER2 expressed on the DCIS cells in the subject (HER2-CAR-T cells).

T-cells are isolated from peripheral blood of enrolled human subjects with DCIS by immuno-affinity-based enrichment and the cells are cultured and transduced with a vector including a death gene safety switch iC9 attached to the HER2scFV-CD8a hinge-CD28Tm-CD28-CD3ζ construct described by Sun et al. (Breast Cancer Research, 2014, 16:R61). The modified cells expressing HER2-CAR (iC9- HER2scFV-CD8a hinge-CD28Tm-CD28-CD3ζ) are cryopreserved in medium in individual cannula(e), each containing single unit dose of the cells, which is about 1×10⁵ cells/kg body weight, 1×10⁶ cells/kg body weight, and 1×10⁷ cells/kg body weight of the subject. The cells are maintained at a temperature below -130°C prior to transpapillary delivery.

Subjects are preconditioned at days -7, -2 with cyclophosphamide (dose ranging between 40 mg/kg and 80 mg/kg body weight of the subject) prior to administration of HER2-CAR-T cells.

Just prior to initiation of cell therapy on day 0, blood are obtained from the subjects and, optionally, the levels of one or more serum factors indicative of cytokine release syndrome (CRS) such as TNF-α, interfcron-γ, and IL-6, are determined in the serum by ELISA. Tumor burden is optionally assessed by measurement of the size or mass of a carcinoma, such as by PET or CT scan or by measuring the numbers of cells of the patient associated with the cancer such as in nipple aspirate or ductal lavage fluid before treatment starts. An MRI scan of subjects' breasts and torso is performed to establish a baseline for later determination of extent of migration of the administered cells.

The cells are thawed at the bedside by wanning to approximately 37°C. The subject's breast nipples are prepared as described in Example 1, and wiped with alcohol wipes and keratin plug (if any) from the mammary papilla of the affected breast is removed. The subjects are then administered a dose of the cells in approximately 1 mL volume by transpapillary administration of the cells into the lumen of a breast duct over a period of 5 to 15 mins. For some subjects, the amount of the dose is a single unit dose. For other subjects, the dose is a split unit dose. This is desirable where there is need for volume restriction. Such a split unit dose of 5×10⁴ cells/kg body weight in two aliquots is administered over 2 days. For subjects deemed to have low tumor burden, more than one single unit dose may be delivered, for example, additional single unit dose.

Following the administration of the dose, the subject is physically examined and monitored for fever, hypoxia, and neurological disturbances. Blood samples are drawn periodically over the next 36 hours to determine the levels of cytokines, c-reactive protein (CRP) and other serum factors and these levels are compared with the levels observed prior to the administration of the dose of modified cells expressing HER-2 CAR. If the post-dose levels are higher than pre-dose levels, depending on the severity of the side effects or CRS, subject is administered anti-IL6 therapy or a CID molecule such as AP1903 or AP20187.

The migration and/or tumor infiltration by the administered cells is determined by one or more MRI scans, for example at 1, 2, 3, and/or 4 weeks after dosing the subject. The percent reduction in tumor burden achieved by such cells is measured by PET, CT or MRI scan.

The presence or absence of an anti-CAR immune response in the subject is optionally detected following the administration of the cells, for example, at 1, 2, 3, or 4 weeks following the administration, for example by, detecting for the presence of the antibodies directed to the CAR by immunostaining of incisional or excisional biopsy specimens taken from the breast.

Some subjects receive a first dose as described above followed by one or more subsequent doses of modified cells expressing HER2-CAR. The size of the subsequent doses is patient-specific. In some subjects, a subsequent dose of 1×10⁶ cells/kg body weight of the subject is administered transpapillarily into the subject's breast duct 3 weeks following the initiation of the first dose, over approximately 10 - 30 min. Subjects are monitored after the first dose and monitoring may continue for several years for any toxic outcomes, and/or recurrence of DCIS. Optionally, some subjects may receive a second subsequent dose of 1×10⁷ cells/kg body weight.

Development of host immune response to HER2-CAR expressing cells and/or CRS, MAS, TLS, neutotoxicity, etc., is assessed. Migration of administered cells to the tumor will be assessed by MRI and tumor burden will be determined.

In event of a subject having a CRS-related event, subject will be administered or a CID molecule such as AP1903 or AP20187 to switch of the HER2-CAR expression and eliminate HER2-CAR-expressing modified cells. Anti-IL-6 therapy, such as Tocilizumab (Actemra), Atizumab (RoActemra) may also be administered depending on the severity of the CRS-related event.

While illustrative embodiments have been illustrated and described, it will be appreciated that various changes can be made therein without departing from the spirit and scope of the invention.

### CLAUSES

1. A transpapillary method of adoptive cell therapy for treatment of a subject having or at risk of having a breast disorder comprising administering cells into a breast duct of the subject.
2. The method of Clause 1, wherein the cells comprise a T-cell, an NK cell, a CTL, a TIL, a monocyte, a granulocyte, or progenitors thereof.
3. The method of Clause 1, wherein the cells comprise modified cells expressing one or more recombinant receptors that bind a target antigen on a breast cell.
4. The method of Clause 3, wherein the one or more recombinant receptors comprises a chimeric antigen receptor (CAR) or an engineered or disease-specific TCR.
5. The method of Clause 3, wherein the modified cells express two or more CARs.
6. The method of any one of Clauses 3 to 5, wherein the one or more recombinant receptors are independently monospecific, bispecific, or multispecific.
7. The method of any one of Clauses 3 to 6, wherein the one or more recombinant receptors is/are constitutively, transiently or switchably expressed, or conditionally active.
8. The method any one of Clauses 3 to 7, wherein the modified cells comprise a safety switch selected from the group consisting of death gene switches, a FITC-based switches, and a PNE-based switches.
9. The method of Clause 8, wherein the death gene switch is a HSV-tk, an iCaspase9, or a FADD.
10. The method of any one of Clauses 3 to 9, wherein the target antigen is a tumor specific antigen, a tumor associated antigen, a multi-lineage tumor associated antigen, an oncofetal antigen, a neoantigen, or an immunosuppressive antigen.
11. The method of any one of Clauses 3 to 10, wherein the target antigen is selected from the group consisting of transformation-related molecules such as MUCs, such as MUC1, c-met, cytokeratins such as CK5, CK6, CK14, CK7, CK8, CK14, CK17, CK18, CK19, p53, glycosides, Tn, TF, and sialyl Tn (STn), Lewis x, Lewis a, Lewis y, and gangliosides such as GM3, GD3, 9-0-acetyl GD3, 9-0-acetyl GT3, and N-glycoly-GM3, Folate Receptor alpha, ROR1, neoantigens, tumor-specific antigens and oncofetal antigens, tumor associated antigens such as carcinoembryonic antigen (CEA), L1 cell adhesion molecule (L1CAM), CAFs-related proteins such as fibroblast activation protein (FAP), FAP-α, FSP-1/S100A4, and PDGFR-β, diganglioside GD2, mesothelin, IL-13 receptor IL13R, IL-13 receptor α, ephrinB2, IGFR1, ELIGHT, WT1, TAG-72, Ep-CAM, LFA-1, EGFR, estrogen receptor (ER), progesterone receptor, MAGE1, MAGE-3, MAGE-A3/6, MAGE-A family members such as MAGE-A1, MAGE-A2, MAGE-A3, MAGE-A4, MAGE-A6, MAGE-A 12, MAGE-A9, MAGE-A11, MAGE-C1, and MAGE-C2, RAGE, BCR-ABL, protein tyrosine kinases such as PRL-2 and PRL3, tumor associated glycoproteins such as TAG-72, CA 19-9, CA 27.29, CA 72-4, CA 50, PD-1, CTLA-4, CD47, receptor tyrosine kinases such as H4-RET, Ki-67, cyclin D1, cyclin A, cyclin E, p16, p21, p27, p53, Bcl-2, Bax, survivin, c-myc, Rb, VEGF, HPR1, HER1, HER2, HER3, HER4, CD10, SPARC, COX-2, basal cytokeratins, CK5/6, CK14, and CK 17, epidermal growth factor receptor, c-kit, c-erbB-2, IL-10, TGF-beta, CCL17, CCL22, and CCL24 stroma released factors such as EGF, HGF, MCP-1, CSF-1, VEGF, cytokines such as IL1, IL-8, TNF-alpha, enzymes such as MM2, MMP7, MMP8, MMP9, MMP12, MMP13, and COX2.
12. The method of any one of Clauses 3 to 11, wherein the recombinant receptor is a HER2-CAR, FR-α-CAR, or a FAP-CAR.
13. The method of any one of Clauses 3 to 12, wherein the recombinant receptor comprises a primary signaling molecule selected from the group consisting of TCR zeta, FcR gamma, FcR beta, CD3 gamma, CD3 delta, CD3 epsilon, CD4, CD8, CD16, CD22, CD25, CD79a, CD79b, and CD66d.
14. The method of any one of Clauses 3 to 13, wherein the recombinant receptor comprises one or more co-stimulatory molecules.
15. The method of any one of Clauses 3 to 14, wherein the co-stimulatory molecule is selected from the group consisting of MHC class I molecule, BTLA and a Toll ligand receptor, immunoglobulin superfamily (IgSF) such as CD28, B7 receptor family members (B7-H2/B7RP-1/LICOS/GL50, B7-DC/PD-L2, B7-H3), CD226, TIM, CD2/SLAM, BTN, LAIR, tumor necrosis factor receptor superfamily (TNFRSF) such as OX40, CD27, CD30, DR3, GITR, and HVEM, CD2 and SLAM on T-cells, ICAM-1, LFA-1 (CD11a/CD18), adhesion molecules (CD54, CD58, CD70), ICOS, CD40, CD40L, 4-1BB (CD137), CD70, CD80, CD86, DAP10, and other orphan receptor families such as LAG3 (CD223) and CD160.
16. The method of any one of Clauses 3 to 14, wherein the recombinant receptor comprises CD28 and 4-1BB as co-stimulatory molecules.
17. The method of any one of Clauses 3 to 16, wherein the modified cells comprise a T-cell, an NK cell, a CTL, a monocyte, a granulocyte, or progenitors thereof.
18. The method of any one of Clauses 3 to 17, wherein the modified cells further
   comprise a dye or a contrasting agent selected from the groups consisting of gadolinium chelates, superparamagnetic iron oxide nanoparticles (SPION), ¹⁹F perfluorocarbon nanoparticles, and other magnetic reporter genes, such as metalloprotein-based MRI probes.
19. The method of any one of the preceding clauses, wherein the cells are formulated in a composition further comprising a pharmaceutically acceptable carrier, buffer, an excipient or a combination thereof.
20. The method of Clause 19, wherein the carrier is lactated Ringers Solution.
21. The method of Clause 19, wherein the composition further comprises a gelling agent.
22. The method according to any one of the preceding clauses, wherein the breast disorder is benign breast disease, breast cancer, Paget's disease of the nipple, or phyllodes tumor.
23. The method according to any one of the preceding clauses, wherein the breast disorder is hyperplasia, atypia, ductal hyperplasia, lobular hyperplasia, atypical ductal hyperplasia (ADH), or atypical lobular hyperplasia (ALH).
24. The method according to any one of the preceding clauses, wherein the breast disorder is a breast cancer selected from the group consisting of ductal carcinoma in situ (DCIS), lobular carcinoma in situ (LCIS), invasive (or infiltrating) lobular carcinoma (ILC), invasive (or infiltrating) ductal carcinoma (IDC), microinvasive breast carcinoma (MIC), inflammatory breast cancer, ER-positive (ER+) breast cancer, progesterone receptor positive (PR+) breast cancer, ER+/PR+ breast cancer, ER-negative (ER-) breast cancer, HER2+ breast cancer, triple negative breast cancer (i.e., ER-/PR-/Her2-breast cancer; "TNBC"), adenoid cystic (adenocystic) carcinoma, low-grade adenosquamatous carcinoma, medullary carcinoma, mucinous (or colloid) carcinoma, papillary carcinoma, tubular carcinoma, metaplastic carcinoma, or micropapillary carcinoma.
25. The method according to any one of the preceding clauses, wherein the breast cancer is a pre-cancer, an early stage cancer, a non-metastatic cancer, a pre-metastatic cancer, a locally advanced cancer, a metastatic cancer or a recurrent cancer.
26. The method according to any one of the preceding clauses, wherein the cells are administered in a single dose or multiple doses.
27. The method of Clause 26, wherein the multiple doses comprise a first dose and one or more subsequent doses.
28. The method of Clause 26, wherein one or more dose is administered in a split unit dose.
29. The method according to any one of the preceding clauses, wherein the subject is administered a unit dose of cells ranging from 1×10³ to 1×10⁹ modified cells/kg body weight, from 1×10³ to 5×10⁸ modified cells/kg body weight, from 0.5×10³ to 1×10⁷ modified cells/kg body weight, from 1×10⁴ to 0.5×10⁶ modified cells/kg body weight, from 0.5×10⁴ to 1×10⁶ modified cells/kg body weight, from 1×10⁵ to 0.5×10⁶ modified cells/kg body weight.
30. The method of clause 27, wherein the first dose is a low dose, such as less than 1×10³ cells/kg, less than 0.5×10⁴ cells/kg, less than 1×10⁴ cells/kg, less than 0.5×10⁵ cells/kg, less than 1×10⁵ cells/kg, less than 0.5×10⁶ cells/kg, or less than 1×10⁶ cells/kg.
31. The method of clause 27, wherein the first dose is a high dose, such as greater than 0.5×10⁵ cells/kg, greater than 1×10⁵ cells/kg, greater than 0.5×10⁶ cells/kg, greater than 1×10⁶ cells/kg, greater than 0.5×10⁷ cells/kg, greater than 1×10⁷ cells/kg, greater than 0.5×10⁸ cells/kg, greater than 1×10⁸ cells/kg, or greater than 5×10⁸ cells/kg.
32. The method of Clause 27, wherein a subsequent dose is administered between 7 and 28 days after the initiation of the first dose.
33. The method of Clause 27, wherein a subsequent dose is same as the first dose, lower than the first dose, or higher than the first dose.
34. The method according to any one of the preceding clauses, wherein the cells are administered as primary therapy, neoadjuvant therapy, or adjuvant therapy.
35. The method according to any one of the preceding clauses, wherein the administration of cells reduces disease burden of the breast disorder in the subject.
36. The method according to any one of the preceding clauses, the administration of modified cells comprising a recombinant receptor reduces tumor burden in a subject.
37. The method according to any one of the preceding clauses, wherein administration of cells reduces tumor burden.
38. The method according to any one of the preceding clauses, wherein administration of modified cells reduces tumor burden.
39. The method according to any one of the preceding clauses, wherein administration reduces a risk of a CRS-related, a MAS-related, a TLS-related, a neurotoxicity-related or a host immune response-related outcome.
40. The method according to any one of the preceding clauses, wherein the administration of cells reduces circulating or breast tissue levels of cytokines such as IFNγ, TNFα, IL-2, GM-CSF, IL-lbeta, IL-6, IL-7, IL-8, IL-10, IL-12, Flt-3, fractalkine, MIP1, sIL-2Rα, and IL-5.
41. The method according to any one of the preceding clauses, wherein the administration of modified cells reduces circulating or breast tissue levels of cytokines such as IFNγ, TNFα, IL-2, GM-CSF, IL-lbeta, IL-6, IL-7, IL-8, IL-10, IL-12, Flt-3, fractalkine, MIP1, sIL-2Rα, and IL-5.
42. The method according to any one of the preceding clauses, wherein the subject is preconditioned with a lymphodepleting agent or a chemotherapeutic agent prior to administration of the cells.
43. The method according to Clause 42, wherein the lymphodepleting agent or a chemotherapeutic agent is selected from the group consisting of cyclophosphamide, cyclosporine, fludarabine, bendamustine, lenalidomide, pomalidomide, gemcitabine, BTK inhibitors such as ibrutinib, oncolytic adenovirus or combinations thereof.
44. The method of any one of the preceding clauses, wherein the subject is administered an additional therapeutic agent or therapy.
45. The method of Clause 44, wherein the additional therapeutic agent is selected from the group consisting of asparaginase, busulfan, carboplatin, cisplatin, daunorubicin, doxorubicin, fluorouracil, gemcitabine, hydroxyurea, methotrexate, paclitaxel, rituximab, vinblastine, vincristine, oncolytic viruses such as oncolytic adenovirus, anti-estrogens such as tamoxifen, N-methyl-endoxifen, nor-endoxifen, endoxifen, raloxifen, fulvestrant and/or aromatase inhibitors such as anastrozole, letrozole, and exemestane.
46. A transpapillary method of adoptive cell therapy for treatment of a subject having or at risk of having a breast disorder comprising administering modified cells expressing a HER2-CAR, a FAP-CAR, or a FR-α into a breast duct of the subject.
47. The method of Clause 46, wherein the HER2-CAR has at least 80%, at least 81%, at least 82%, at least 83%, at least 84%, at least 85%, at least 86%, at least 87%, at least 88%, at least 89%, at least 90%, at least 91%, at least 92%, at least 93%, at least 93%, at least 94%, at least 95%, at least 96%, at least 97%, at least 98%, at least 99%, and 100% homology to SEQ ID NO:1 disclosed in FIGURE 2 or a variant or functional portion thereof.
48. The method of Clause 46, wherein the FAP-CAR has at least 80%, at least 81%, at least 82%, at least 83%, at least 84%, at least 85%, at least 86%, at least 87%, at least 88%, at least 89%, at least 90%, at least 91%, at least 92%, at least 93%, at least 93%, at least 94%, at least 95%, at least 96%, at least 97%, at least 98%, at least 99%, and 100% homology to SEQ ID NO:2 disclosed in FIGURE 2 or a variant or functional portion thereof.
49. An article of manufacture, comprising one or more containers, packaging material, a label or package insert, and, optionally, a device.
50. The article of manufacture of Clause 49, wherein the device is a needle and syringe, a cannula, a catheter, a microcatheter, an osmotic pump, or an encapsulation device.

## Claims

1. Cells comprising modified lymphocyte cells expressing one or more recombinant receptors that bind a target antigen on a breast cancer cell for use in a method of treating or preventing breast cancer in a subject the method comprising intraductally administering said cells into a breast duct of the subject, wherein the target antigen is a breast cancer-specific antigen, a breast cancer-associated antigen, a multi-lineage breast cancer-associated antigen, an oncofetal breast cancer-antigen, a breast cancer neoantigen, or an immunosuppressive breast cancer antigen.

2. The cells for use according to Claim 1, wherein the target antigen is a breast cancer-specific antigen.

3. The cells for use according to any one of the preceding Claims, wherein the target antigen is selected from the group consisting of transformation-related molecules such as MUCs, such as MUC1, c-met, cytokeratins such as CK5, CK6, CK14, CK7, CK8, CK17, CK18, CK19, p53, glycosides, Tn, TF, and sialyl Tn (STn), Lewis x, Lewis a, Lewis y, and gangliosides such as GM3, GD3, 9-0-acetyl GD3, 9-0-acetyl GT3, and N-glycoly-GM3, Folate Receptor alpha, ROR1, neoantigens, tumor-specific antigens and oncofetal antigens, tumor associated antigens such as carcinoembryonic antigen (CEA), L1 cell adhesion molecule (L1CAM), CAFs-related proteins such as fibroblast activation protein (FAP), FAP-α, FSP-1/S100A4, and PDGFR-β, diganglioside GD2, mesothelin, IL-13 receptor IL13R, IL-13 receptor α, ephrinB2, IGFR1, ELIGHT, WT1, TAG-72, Ep-CAM, LFA-1, EGFR, estrogen receptor (ER), progesterone receptor, MAGE1, MAGE-3, MAGE-A3/6, MAGE-A family members such as MAGE-A1, MAGE-A2, MAGE-A3, MAGE-A4, MAGE-A6, MAGE-A12, MAGE-A9, MAGE-A11, MAGE-C1, and MAGE-C2, RAGE, BCR-ABL, protein tyrosine kinases such as PRL-2 and PRL3, tumor associated glycoproteins such as TAG-72, CA 19-9, CA 27.29, CA 72-4, CA 50, PD-1, CTLA-4, CD47, receptor tyrosine kinases such as H4-RET, Ki-67, cyclin D1, cyclin A, cyclin E, p16, p21, p27, p53, Bcl-2, Bax, survivin, c-myc, Rb, VEGF, HPR1, HER1, HER2, HER3, HER4, CD10, SPARC, COX-2, basal cytokeratins, CK5/6, CK14, and CK 17, epidermal growth factor receptor, c-kit, c-erbB-2, IL-10, TGF-beta, CCL17, CCL22, and CCL24 stroma released factors such as EGF, HGF, MCP-1, CSF-1, VEGF, cytokines such as IL1, IL-8, TNF-alpha, enzymes such as MM2, MMP7, MMP8, MMP9, MMP12, MMP13, and COX2.

4. The cells for use according to any one of the preceding Claims wherein the modified lymphocyte cells comprise a T-cell, an NK cell, a CTL, a TIL, or progenitors thereof.

5. The cells for use according to any one of the preceding Claims, wherein:
(a) the one or more recombinant receptors comprises a chimeric antigen receptor (CAR) or an engineered or disease-specific TCR; or
(b) the modified lymphocyte cells express two or more CARs.

6. The cells for use according to any one of the preceding Claims, wherein:
(a) the one or more recombinant receptors are independently monospecific, bispecific, or multispecific; and/or
(b) the one or more recombinant receptors is/are constitutively, transiently or switchably expressed, or conditionally active.

7. The cells for use according to any one of the preceding Claims, wherein:
(a) the recombinant receptor is a HER2-CAR, FR-α-CAR, or a FAP-CAR; and/or
(b) the recombinant receptor comprises a primary signaling molecule selected from the group consisting of TCR zeta, FcR gamma, FcR beta, CD3 gamma, CD3 delta, CD3 epsilon, CD4, CD8, CD16, CD22, CD25, CD79a, CD79b, and CD66d; and/or
(c) the recombinant receptor comprises one or more co-stimulatory molecules; optionally wherein:
(i) the co-stimulatory molecule is selected from the group consisting of MHC class I molecule, BTLA and a Toll ligand receptor, immunoglobulin superfamily (IgSF) such as CD28, B7 receptor family members (B7-H2/B7RP-1/LICOS/GL50, B7-DC/PD-L2, B7-H3), CD226, TIM, CD2/SLAM, BTN, LAIR, tumor necrosis factor receptor superfamily (TNFRSF) such as OX40, CD27, CD30, DR3, GITR, and HVEM, CD2 and SLAM on T-cells, ICAM-1, LFA-1 (CD11a/CD18), adhesion molecules (CD54, CD58, CD70), ICOS, CD40, CD40L, 4-1BB (CD137), CD70, CD80, CD86, DAP10, and other orphan receptor families such as LAG3 (CD223) and CD160; and/or
(ii) the recombinant receptor comprises CD28 and 4-1BB as co-stimulatory molecules.

8. The cells for use according to any one of the preceding Claims, wherein:
(a) the modified lymphocyte cells comprise a safety switch selected from the group consisting of death gene switches, a FITC-based switches, and a PNE-based switches; optionally wherein the death gene switch is a HSV-tk, an iCaspase9, or a FADD; and/or
(b) the modified lymphocyte cells comprise a T-cell, an NK cell, a CTL, a monocyte, a granulocyte, or progenitors thereof; and/or
(c) the modified lymphocyte cells further comprise a dye or a contrasting agent selected from the groups consisting of gadolinium chelates, superparamagnetic iron oxide nanoparticles (SPION), ¹⁹F perfluorocarbon nanoparticles, and other magnetic reporter genes, such as metalloprotein-based MRI probes; and/or
(d) the cells are formulated in a composition further comprising a pharmaceutically acceptable carrier, buffer, an excipient or a combination thereof; optionally wherein:
(i) the carrier is lactated Ringers Solution; or
(ii) the composition further comprises a gelling agent.

9. The cells for use according to any one of the preceding Claims, wherein:
(a) the breast cancer is selected from the group consisting of ductal carcinoma in situ (DCIS), lobular carcinoma in situ (LCIS), invasive (or infiltrating) lobular carcinoma (ILC), invasive (or infiltrating) ductal carcinoma (IDC), microinvasive breast carcinoma (MIC), inflammatory breast cancer, ER-positive (ER+) breast cancer, progesterone receptor positive (PR+) breast cancer, ER+/PR+ breast cancer, ER-negative (ER-) breast cancer, HER2+ breast cancer, triple negative breast cancer (i.e., ER-/PR-/Her2-breast cancer; "TNBC"), adenoid cystic (adenocystic) carcinoma, low-grade adenosquamatous carcinoma, medullary carcinoma, mucinous (or colloid) carcinoma, papillary carcinoma, tubular carcinoma, metaplastic carcinoma, or micropapillary carcinoma; and/or
(b) the breast cancer is a pre-cancer, an early stage cancer, a non-metastatic cancer, a pre-metastatic cancer, a locally advanced cancer, a metastatic cancer or a recurrent cancer.

10. The cells for use according to any one of the preceding Claims, wherein the cells are administered in a single dose or multiple doses; optionally wherein:
(a) the multiple doses comprise a first dose and one or more subsequent doses; optionally wherein:
(i) the first dose is a low dose, such as less than 1×10³ cells/kg, less than 0.5×10⁴ cells/kg, less than 1×10⁴ cells/kg, less than 0.5×10⁵ cells/kg, less than 1×10⁵ cells/kg, less than 0.5×10⁶ cells/kg, or less than 1×10⁶ cells/kg;
(ii) the first dose is a high dose, such as greater than 0.5×10⁵ cells/kg, greater than 1×10⁵ cells/kg, greater than 0.5×10⁶ cells/kg, greater than 1×10⁶ cells/kg, greater than 0.5×10⁷ cells/kg, greater than 1×10⁷ cells/kg, greater than 0.5×10⁸ cells/kg, greater than 1×10⁸ cells/kg, or greater than 5×10⁸ cells/kg;
(iii) a subsequent dose is administered between 7 and 28 days after the initiation of the first dose; or
(iv) a subsequent dose is same as the first dose, lower than the first dose, or higher than the first dose;
and/or
(b) one or more dose is administered in a split unit dose.

11. The cells for use according to any one of the preceding Claims, wherein the subject is administered a unit dose of cells ranging from 1×10³ to 1×10⁹ modified lymphocyte cells/kg body weight, from 1×10³ to 5×10⁸ modified lymphocyte cells/kg body weight, from 0.5×10³ to 1×10⁷ modified lymphocyte cells/kg body weight, from 1×10⁴ to 0.5×10⁶ modified lymphocyte cells/kg body weight, from 0.5×10⁴ to 1×10⁶ modified lymphocyte cells/kg body weight, from 1×10⁵ to 0.5×10⁶ modified lymphocyte cells/kg body weight.

12. The cells for use according to any one of the preceding Claims, wherein:
(a) the cells are administered as primary therapy, neoadjuvant therapy, or adjuvant therapy; and/or
(b) the administration of the cells reduces tumor burden in the subject; and/or
(c) the administration of the cells reduces a risk of a cytokine release syndrome (CRS)-related, a macrophage activation syndrome (MAS)-related, a tumor lysis syndrome (TLS)-related, a neurotoxicity-related or a host immune response-related outcome; and/or
(d) the administration of the cells reduces circulating or breast tissue levels of cytokines such as IFNy, TNFα, IL-2, GM-CSF, IL-1beta, IL-6, IL-7, IL-8, IL-10, IL-12, Flt-3, fractalkine, MIP1, sIL-2Rα, and IL-5; and/or
(e) the subject is preconditioned with a lymphodepleting agent or a chemotherapeutic agent prior to administration of the cells; optionally wherein the lymphodepleting agent or a chemotherapeutic agent is selected from the group consisting of cyclophosphamide, cyclosporine, fludarabine, bendamustine, lenalidomide, pomalidomide, gemcitabine, BTK inhibitors such as ibrutinib, oncolytic adenovirus or combinations thereof; and/or
(f) the subject is administered an additional therapeutic agent or therapy; optionally wherein the additional therapeutic agent is selected from the group consisting of asparaginase, busulfan, carboplatin, cisplatin, daunorubicin, doxorubicin, fluorouracil, gemcitabine, hydroxyurea, methotrexate, paclitaxel, rituximab, vinblastine, vincristine, oncolytic viruses such as oncolytic adenovirus, anti-estrogens such as tamoxifen, N-methyl-endoxifen, nor-endoxifen, endoxifen, raloxifen, fulvestrant and/or aromatase inhibitors such as anastrozole, letrozole, and exemestane.

13. The cells for use according to any one of the preceding Claims, wherein said treating or preventing comprises administering cells comprising modified lymphocyte cells expressing a HER2-CAR, a FAP-CAR, or a FR-α into a breast duct of the subject; optionally wherein:
(a) the HER2-CAR has at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, and 100% homology to SEQ ID NO:1 disclosed in FIGURE 2 or a variant or functional portion thereof; or
(b) the FAP-CAR has at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, and 100% homology to SEQ ID NO:2 disclosed in FIGURE 2 or a variant or functional portion thereof.

14. The cells for use according to any one of the preceding Claims, wherein said cells comprise a dye or a contrasting agent; the method further comprising monitoring the administered cells for migration, tumor infiltration, or appearance in blood or other tissues; optionally wherein the dye or a contrasting agent is selected from the group consisting of gadolinium chelates, superparamagnetic iron oxide nanoparticles (SPION), ¹⁹F perfluorocarbon nanoparticles, and other magnetic reporter genes, such as metalloprotein-based MRI probes.
